# EUROPEAN PATENT SPECIFICATION

(11) **EP 3 769 087 B1**
(45) Date of publication and mention of the grant of the patent: **27.09.2023**
(21) Application number: 19713756.5
(22) Date of filing: 22.03.2019
(51) Int. Cl.: G01N 33/68

(54) **A METHOD FOR DIAGNOSING METABOLIC DISORDER**
VERFAHREN ZUR DIAGNOSE VON STOFFWECHSELERKRANKUNGEN
PROCÉDÉ DE DIAGNOSTIC D'UN TROUBLE MÉTABOLIQUE

(30) Priority: 23.03.2018 GB 201804734
(43) Date of publication of application: 27.01.2021
(73) Proprietor: University College Dublin, National University of Ireland, Dublin, Dublin 4 (IE)
(72) Inventor: MCGILLICUDDY, Fiona, Dublin, 16 (IE); PENNINGTON, Stephen, Nr Whitegate Co Clare, V94 W5TV (IE)
(74) Representative: FRKelly
(86) International application number: PCT/EP2019/057347
(87) International publication number: WO 2019/180264

(56) References cited:
- A.I. RUPÉREZ ET AL: "Cardiovascular risk biomarkers and metabolically unhealthy status in prepubertal children: Comparison of definitions", NMCD. NUTRITION METABOLISM AND CARDIOVASCULAR DISEASES, vol. 28, no. 5, 16 February 2018 (2018-02-16), pages 524-530, XP055595181, IT ISSN: 0939-4753, DOI: 10.1016/j.numecd.2018.02.006
- PETRI K WIKLUND ET AL: "Serum metabolic profiles in overweight and obese women with and without metabolic syndrome", DIABETOLOGY & METABOLIC SYNDROME, BIOMED CENTRAL LTD, LONDON, UK, vol. 6, no. 1, 20 March 2014 (2014-03-20), page 40, XP021183966, ISSN: 1758-5996, DOI: 10.1186/1758-5996-6-40
- SCOTT AHL ET AL: "Adiponectin Levels Differentiate Metabolically Healthy vs Unhealthy Among Obese and Nonobese White Individuals", JOURNAL OF CLINICAL ENDOCRINOLOGY AND METABOLISM, vol. 100, no. 11, 1 November 2015 (2015-11-01), pages 4172-4180, XP055595176, US ISSN: 0021-972X, DOI: 10.1210/jc.2015-2765
- SUZANNE E KINGERY ET AL: "Gene CNVs and protein levels of complement C4A and C4B as novel biomarkers for partial disease remissions in new-onset type 1 diabetes patients : Complement C4 in diabetes partial remission", PEDIATRIC DIABETES, vol. 13, no. 5, 1 August 2012 (2012-08-01) , pages 408-418, XP055595177, DK ISSN: 1399-543X, DOI: 10.1111/j.1399-5448.2011.00836.x
- M COPENHAVER ET AL: "Complement C3 and C4 and Metabolic Syndrome", ANNALS OF DIABETES RESEARCH, vol. 1, no. 1, 10 August 2017 (2017-08-10) , XP055595178,
- B. BLASKO ET AL: "Low complement C4B gene copy number predicts short-term mortality after acute myocardial infarction", INTERNATIONAL IMMUNOLOGY, vol. 20, no. 1, 20 November 2007 (2007-11-20), pages 31-37, XP055595179, ISSN: 0953-8178, DOI: 10.1093/intimm/dxm117

## Description

### Field of the Invention

The present invention relates to a method of diagnosing or prognosing a metabolic disorder selected from one or more of abdominal obesity, high blood pressure (hypertension), high blood sugar (hyperglycaemia), high serum triglycerides (hypertriglyceridemia), and low high-density lipoprotein (HDL) levels (HDL-cholesterol(C)) in a subject.

### Background to the Invention

Obesity is a global public health epidemic and an independent risk factor for the development of cardiovascular disease (CVD). The mechanisms linking these disease states however are not fully understood. Presence of other co-morbidities including insulin resistance (IR), hypertension and type 2 diabetes mellitus further increases CVD risk in obese individuals. Obesity often co-exists with the metabolic syndrome (MetS) which is diagnosed as the clustering of three or more of the following; abdominal obesity, hyperglycaemia, hypertension, hypertriglyceridemia, and reduced HDL-cholesterol(C)) according to the Adult Treatment Program III criteria. Approximately 1 0-40% of obese individuals present with no cardiometabolic abnormalities and are classified as metabolically healthy but obese (MHO) while those with more metabolic complications are classified as metabolically unhealthy obese (MUO).

Metabolic dyslipidemia is a classic hallmark of obesity with increased levels of small, dense low-density lipoprotein (LDL) particles and circulating triacylglyerides (TAG) and reduced levels of HDL-C. While HDL-C is inversely associated with CVD, raising HDL-C levels using cholesterol ester transfer protein (CETP) inhibitors failed to have clinical benefit in high risk patients. Furthermore genetically-defined low HDL-C has not been associated with CVD. These findings have questioned the role of HDL particles during CVD pathogenesis and have highlighted the need for a greater understanding of HDL particle biology, particularly in the disease setting.

Reliance on static HDL-C measurements to reflect HDL particle biology is particularly over-simplified. HDL particles are heterogeneous complex particles which differ according to size, density, charge, lipid and protein composition and in turn function, including anti-oxidant, anti-inflammatory, anticoagulant, anti-thrombotic and cholesterol-efflux promoting functions. Small HDL particles mediate cholesterol efflux via the ATP-binding cassette (ABC), sub-family A, member 1 (ABCA1) transporter (ABCA1-dependent efflux), while larger particles mediate efflux via ABC sub-family G member 1 (ABCG1) and scavenger receptor class B member 1 (SR-BI) transporters (ABCA1-independent efflux). Measurement of HDL efflux capacity in turn is a better predictor of CVD than static HDL-C and similarly ABCA1 -dependent efflux, but not HDL-C, inversely correlates with pulse wave velocity in humans.

SUZANNE E KINGERY ET AL, "Gene CNVs and protein levels of complement C4A and C4B as novel biomarkers for partial disease remissions in new-onset type 1 diabetes patients : Complement C4 in diabetes partial remission", PEDIATRIC DIABETES,Vol. 13, No. 5, 01 August 2012 (2012-08-01), page 408-418 discloses C4A appears to associate with the protection of residual beta-cell function in new-onset T1D and C4B is correlated with the end of disease remission at 9-mont post diagnosis.

M COPENHAVER ET AL, "Complement C3 and C4 and Metabolic Syndrome", ANNALS OF DIABETES RESEARCH,Vol. 1, No. 1, 10 August 2017 (2017-08-10) discloses adipose tissue not only produces complement but is also a potential target for complement activity, and obese adults and adults with cardiometabolic disease who are not obese have been shown to have increased complement levels.

B. BLASKO ET AL, "Low complement C4B gene copy number predicts short-term mortality after acute myocardial infarction", INTERNATIONAL IMMUNOLOGY,Vol. 20, No. 1, 20 November 2007 (2007-11-20), page 31-37 discloses the C4B*Q0 genotype was found to be strongly associated with 1-year mortality with a hazard ratio of 3.50, which association was restricted to ever-smoking patients, whereas neither C4A gene copy number nor LTA252 SNP did confer increased risk of mortality after AMI.

There is a need for an improved method of diagnosing or prognosing a metabolic disorder in a subject, and which does not rely on static HDL-C measurements as a reflection of HDL particle biology.

### Summary of the Invention

According to a first aspect of the present invention, there is provided a method of diagnosing or prognosing a metabolic disorder selected from one or more of abdominal obesity, high blood pressure (hypertension), high blood sugar (hyperglycaemia), high serum triglycerides (hypertriglyceridemia), and low high-density lipoprotein 5 (HDL) levels (HDL-cholesterol(C)) in a subject, the method comprising the steps of:
(a) determining the quantitative or qualitative level of the protein biomarkers in a biological sample from the subject; and
(b) diagnosing or prognosing the metabolic disorder in the subject based on the quantitative or qualitative level of each protein biomarker in the biological sample;
wherein the protein biomarkers comprise Complement C4-B; Heparin cofactor 2; Protein IGKV2D-28; Apolipoprotein C-I; Apolipoprotein C-III; Ig heavy chain V-III region 23; Apolipoprotein A-I; Apolipoprotein A-IV; Alpha-2-antiplasmin; and Gelsolin.

The determining step (a) comprises determining the quantitative or qualitative level of each of the biomarkers in the biological sample from the subject.

Each biomarker is a protein. Optionally, each biomarker is a peptide. Further optionally, each biomarker is a polypeptide.

Optionally, the protein biomarkers further comprise Complement factor I; Protein IGHV4-31;
Ceruloplasmin; Serum paraoxonase/arylesterase 1; Apolipoprotein D; Ig lambda-3 chain C regions; Immunoglobulin lambda variable 2-8; Complement C2; Inter-alpha-trypsin inhibitor heavy chain H4; Ig kappa chain C region; Inter-alpha-trypsin inhibitor heavy chain H2; Ig gamma-2 chain C region; Uncharacterized protein B4E1Z4; Protein IGKV1-17; and C-reactive protein cleaved into: C-reactive protein(1-205).

Optionally, the protein biomarkers comprise: Complement C4-B; Heparin cofactor 2; Protein IGKV2D-28; Apolipoprotein C-I; Apolipoprotein C-III; Ig heavy chain V-III region 23; Apolipoprotein A-I; Apolipoprotein A-IV; Alpha-2-antiplasmin; Gelsolin; Complement factor I; Protein IGHV4-31; Ceruloplasmin; Serum paraoxonase/arylesterase 1; Apolipoprotein D; Ig lambda-3 chain C regions; I Immunoglobulin lambda variable 2-8; Complement C2; Inter-alpha-trypsin inhibitor heavy chain H4; Ig kappa chain C region; Inter-alpha-trypsin inhibitor heavy chain H2; Ig gamma-2 chain C region; Uncharacterized protein B4E1Z4; Protein IGKV1-17; and C-reactive protein cleaved into: C-reactive protein(1-205).

Optionally, the protein biomarkers further comprise Leucine-rich alpha-2-glycoprotein; Complement component C6; Coagulation factor X; Protein IGKV1-33; Inter-alpha-trypsin inhibitor heavy chain H1; Sex hormone-binding globulin; Ig heavy chain V-I region 5; Serum amyloid P-component; Vitamin K-dependent 10 protein S; Ig gamma-1 chain C region; Phosphatidylinositol-glycan-specific phospholipase D; Plasma protease C1 inhibitor; Ig lambda chain V-I region 51;Actin, cytoplasmic 2; Protein IGHV3-74; Corticosteroid-binding globulin; Protein IGKV2D-29; Vitamin K-dependent protein C; and Ig lambda chain V-II region BUR.

Optionally, the protein biomarkers comprise: Complement C4-B; Heparin cofactor 2; Protein IGKV2D-28; Apolipoprotein C-I; Apolipoprotein C-III; Ig heavy chain V-III region 23; Apolipoprotein A-I; Apolipoprotein A-IV; Alpha-2-antiplasmin; Gelsolin; Complement factor I; Protein IGHV4-31; Ceruloplasmin; Serum paraoxonase/arylesterase 1; Apolipoprotein D; Ig lambda-3 chain C regions; Immunoglobulin lambda variable 2-8; Complement C2; Inter-alpha-trypsin inhibitor heavy chain H4; Ig kappa chain C region; Inter-alpha-trypsin inhibitor heavy chain H2; Ig gamma-2 chain C region; Uncharacterized protein B4E1Z4; Protein IGKV1-17; C-reactive protein cleaved into: C-reactive protein(1-205); Leucine-rich alpha-2-glycoprotein; Complement component C6; Coagulation factor X; Protein IGKV1-33; Inter-alpha-trypsin inhibitor heavy chain H1; Sex hormone-binding globulin; Ig heavy chain V-I region 5; Serum amyloid P-component; Vitamin K-dependent 10 protein S; Ig gamma-1 chain C region; Phosphatidylinositol-glycan-specific phospholipase D; Plasma protease C1 inhibitor; Ig lambda chain V-I region 51;Actin, cytoplasmic 2; Protein IGHV3-74; Corticosteroid-binding globulin; Protein IGKV2D-29; Vitamin K-dependent protein C; and Ig lambda chain V-II region BUR.

Optionally, the diagnosing or prognosing step (b) comprises comparing the quantitative or qualitative level of each biomarker in the biological sample from the subject with the quantitative or qualitative level of each respective biomarker in a normal sample.

Optionally, the normal sample is a biological sample from a subject not suffering from a metabolic disorder.

Optionally, a quantitative or qualitative level of each biomarker in the biological sample from the subject greater than the quantitative or qualitative level of each respective biomarker in a normal sample is indicative of the quantitative or qualitative level of the metabolic disorder.

Optionally, a quantitative or qualitative level of each biomarker in the biological sample from the subject greater than the quantitative or qualitative level of each respective biomarker in a normal sample is indicative of the quantitative or qualitative presence of the metabolic disorder.

Optionally, the normal sample is a biological sample from a subject not suffering from a metabolic disorder.

Optionally, the biological sample is selected from whole blood, serum, plasma, urine, interstitial fluid, peritoneal fluid, cervical swab, tears, saliva, buccal swab, skin, brain tissue, and cerebrospinal fluid.

Optionally, the biological sample comprises liporotein. Further optionally, the biological sample comprises high-density lipoprotein (HDL). Still further optionally, the biological sample comprises high-density lipoprotein (HDL) particles.

The metabolic syndrome is selected from one or more of abdominal obesity, high blood pressure (hypertension), high blood sugar (hyperglycaemia), high serum triglycerides (hypertriglyceridemia), and low high-density lipoprotein (HDL) levels (HDL-cholesterol(C)).

Optionally, there is provided a method of diagnosing or prognosing a metabolic disorder in a subject, wherein the metabolic syndrome is selected from one or more of abdominal obesity, high blood pressure (hypertension), high blood sugar (hyperglycaemia), high serum triglycerides (hypertriglyceridemia), and low high-density lipoprotein (HDL) levels (HDL-cholesterol(C)); the method comprising the steps of:
(a) determining the quantitative or qualitative level of the protein biomarkers in a biological sample from the subject; and
(b) diagnosing or prognosing the metabolic disorder in the subject based on the quantitative or qualitative level of each protein biomarker in the biological sample;
wherein the protein biomarkers comprise Complement C4-B; Heparin cofactor 2; Protein IGKV2D-28; Apolipoprotein C-I; Apolipoprotein C-III; Ig heavy chain V-III region 23; Apolipoprotein A-I; Apolipoprotein A-IV; Alpha-2-antiplasmin; Gelsolin; Complement factor I; Protein IGHV4-31; Ceruloplasmin; Serum paraoxonase/arylesterase 1; Apolipoprotein D; Ig lambda-3 chain C regions; Immunoglobulin lambda variable 2-8; Complement C2; Inter-alpha-trypsin inhibitor heavy chain H4; Ig kappa chain C region; Inter-alpha-trypsin inhibitor heavy chain H2; Ig gamma-2 chain C region; Uncharacterized protein B4E1Z4; Protein IGKV1-17; C-reactive protein cleaved into: C-reactive protein(1-205); Leucine-rich alpha-2-glycoprotein; Complement component C6; Coagulation factor X; Protein IGKV1-33; Inter-alpha-trypsin inhibitor heavy chain H1; Sex hormone-binding globulin; Ig heavy chain V-I region 5; Serum amyloid P-component; Vitamin K-dependent 10 protein S; Ig gamma-1 chain C region; Phosphatidylinositol-glycan-specific phospholipase D; Plasma protease C1 inhibitor; Ig lambda chain V-I region 51;Actin, cytoplasmic 2; Protein IGHV3-74; Corticosteroid-binding globulin; Protein IGKV2D-29; Vitamin K-dependent protein C; and Ig lambda chain V-II region BUR.

Optionally, there is provided a method of diagnosing or prognosing the selected metabolic disorder in the subject, irrespective of the weight of the subject; the method comprising the steps of:
(a) determining the quantitative or qualitative level of the protein biomarkers in a biological sample from the subject; and
(b) diagnosing or prognosing the metabolic disorder in the subject based on the quantitative or qualitative level of each protein biomarker in the biological sample;
wherein the protein biomarkers comprise Complement C4-B; Heparin cofactor 2; Protein IGKV2D-28; Apolipoprotein C-I; Apolipoprotein C-III; heavy chain V-III region 23; Apolipoprotein A-I; Apolipoprotein A-IV; Alpha-2-antiplasmin; Gelsolin; Complement factor I; Protein IGHV4-31; Ceruloplasmin; Serum paraoxonase/arylesterase 1; Apolipoprotein D; Ig lambda-3 chain C regions; Immunoglobulin lambda variable 2-8; Complement C2; Inter-alpha-trypsin inhibitor heavy chain H4; Ig kappa chain C region; Inter-alpha-trypsin inhibitor heavy chain H2; Ig gamma-2 chain C region; Uncharacterized protein B4E1Z4; Protein IGKV1-17; C-reactive protein cleaved into: C-reactive protein(1-205); Leucine-rich alpha-2-glycoprotein; Complement component C6; Coagulation factor X; Protein IGKV1-33; Inter-alpha-trypsin inhibitor heavy chain H1; Sex hormone-binding globulin; Ig heavy chain V-I region 5; Serum amyloid P-component; Vitamin K-dependent 10 protein S; Ig gamma-1 chain C region; Phosphatidylinositol-glycan-specific phospholipase D; Plasma protease C1 inhibitor; Ig lambda chain V-I region 51;Actin, cytoplasmic 2; Protein IGHV3-74; Corticosteroid-binding globulin; Protein IGKV2D-29; Vitamin K-dependent protein C; and Ig lambda chain V-II region BUR.

| **Biomarker:** | **Name** | **UniProt No** | **SEQ ID NO:** |
|---|---|---|---|
| A2AP | Alpha-2-antiplasmin | P08697 | SEQ ID NO: 1 |
| | Leucine-rich alpha-2- | P02750 | |
| A2GL | glycoprotein | | SEQ ID NO: 2 |
| ACTG | Actin, cytoplasmic 2 | P63261 | SEQ ID NO: 3 |
| APOA1 | Apolipoprotein A-I | P02647 | SEQ ID NO: 4 |
| APOA4 | Apolipoprotein A-IV | P06727 | SEQ ID NO: 5 |
| APOC3 | Apolipoprotein C-III | P02656 | SEQ ID NO: 6 |
| APOD | Apolipoprotein D | P05090 | SEQ ID NO: 7 |
| B4E1Z4 | Uncharacterized protein | B4E1Z4 | SEQ ID NO: 8 |
| CBG | Corticosteroid-binding globulin | P08185 | SEQ ID NO: 9 |
| CERU | Ceruloplasmin | P00450 | SEQ ID NO: 10 |
| CO2 | Complement C2 | P06681 | SEQ ID NO: 11 |
| CO4B | Complement C4-B | P0C0L5 | SEQ ID NO: 12 |
| CO6 | Complement component C6 | P13671 | SEQ ID NO: 13 |
| | C-reactive protein [Cleaved | P02741 | |
| CRP | into: C-reactive protein(1-205)] | | SEQ ID NO: 14 |
| FA10 | Coagulation factor X | P00742 | SEQ ID NO: 15 |
| G3XAM2 | Complement factor I | P05156 | SEQ ID NO: 16 |
| GELS | Gelsolin | P06396 | SEQ ID NO: 17 |
| HEP2 | Heparin cofactor 2 | P05546 | SEQ ID NO: 18 |
| HV304 | Ig heavy chain V-III region 23 | P01765/P01764.2 | SEQ ID NO: 19 |
| I3L145 | Sex hormone-binding globulin | I3L145/P04278.2 | SEQ ID NO: 20 |
| IC1 | Plasma protease C1 inhibitor | P05155 | SEQ ID NO: 21 |
| IGHG1 | Ig gamma-1 chain C region | A0A087WV47/P01857.1 | SEQ ID NO: 22 |
| IGHG2 | Ig gamma-2 chain C region | P01859 | SEQ ID NO: 23 |
| IGHV3-74 | Protein IGHV3-74 | A0A0B4J1X5 | SEQ ID NO: 24 |
| IGHV4-31 | Protein IGHV4-31 | A0A087WSY4 | SEQ ID NO: 25 |
| IGKC | Ig kappa chain C region | A0A087WYL9/P01834.2 | SEQ ID NO: 26 |
| IGKV1-17 | Protein IGKV1-17 | A0A0B4J1Z4/P01599.2 | SEQ ID NO: 27 |
| IGKV1D-33 IGKV1-33 | Protein IGKV1-33 | A0A087WZH9/P01594.2 | SEQ ID NO: 28 |
| IGKV2D-28 | Protein IGKV2D-28 | A0A0A0MTQ6/P01615.2 | SEQ ID NO: 29 |
| IGKV2D-29 | Protein IGKV2D-29 | A0A087X0P6 | SEQ ID NO: 30 |
| IGLC3 | Ig lambda-3 chain C regions | A0A075B6L0/P0DOY3.1 | SEQ ID NO: 31 |
| | Immunoglobulin lambda | A0A087WYR4/P01709.2 | |
| IGLV2-8 | variable 2-8 | | SEQ ID NO: 32 |
| | Inter-alpha-trypsin inhibitor | P19827 | |
| ITIH1 | heavy chain H1 | | SEQ ID NO: 33 |
| | Inter-alpha-trypsin inhibitor | Q14624 | |
| ITIH4 | heavy chain H4 | | SEQ ID NO: 34 |
| K7ERI9 | Apolipoprotein C-I | K7ERI9/P02654.1 | SEQ ID NO: 35 |
| KV110 | Ig heavy chain V-I region 5 | P01602 | SEQ ID NO: 36 |
| LV104 | Ig lambda chain V-I region 51 Ig lambda chain V-II region | P01702 P01708 | SEQ ID NO: 37 |
| LV205 | BUR | | SEQ ID NO: 38 |
| | Phosphatidylinositol-glycan- | P80108 | |
| PHLD | specific phospholipase D | | SEQ ID NO: 39 |
| | Serum | P27169 | |
| PON1 | paraoxonase/arylesterase 1 | | SEQ ID NO: 40 |
| PROC | Vitamin K-dependent protein C | P04070 | SEQ ID NO: 41 |
| PROS | Vitamin K-dependent protein S | P07225 | SEQ ID NO: 42 |
| | Inter-alpha-trypsin inhibitor | Q5T985 | |
| Q5T985 | heavy chain H2 | | SEQ ID NO: 43 |
| SAMP | Serum amyloid P-component | P02743 | SEQ ID NO: 44 |

### Brief Description of the Drawings

Reference will be made to the accompanying drawings in which:

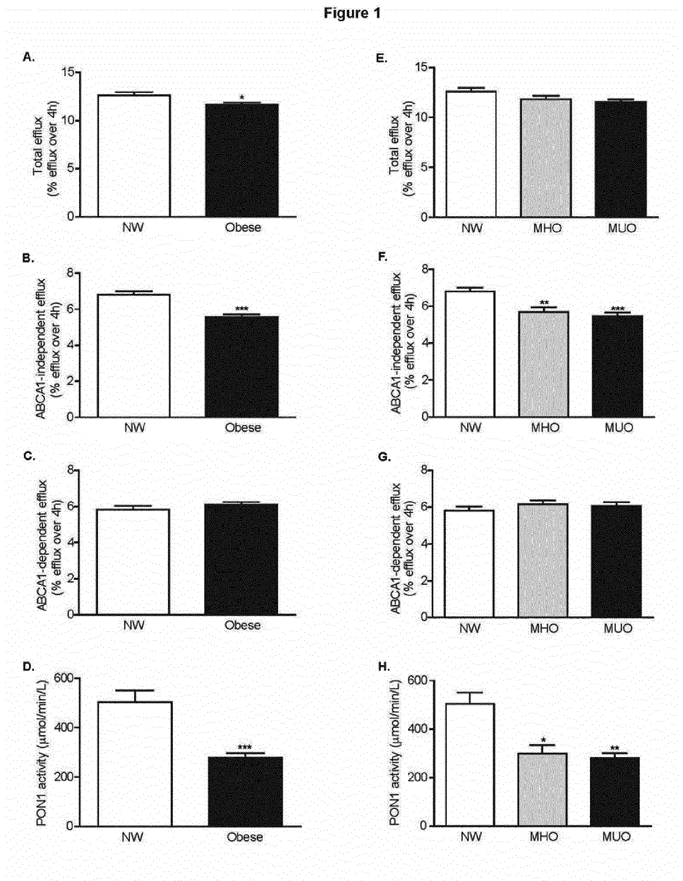

**Figure 1** illustrates serum cholesterol efflux capacity (CEC) and PON1 activity within obese and normal weight (NW) cohorts; wherein J774 macrophages, labelled with ³H-cholesterol (1µCi/ml), were stimulated ± cAMP (0.3mM) to drive ABCA1 protein expression; fasting serum samples were depleted of ApoB particles by PEG precipitation; and percentage ³H-cholesterol efflux to 2.8% PEG-supernatant was monitored over 4h; and wherein (A) Total, (B) ABCA1 -independent and (C) ABCA1-dependent efflux in NW and obese group is presented (NW n=131 , obese *n*=108); and wherein (D) Serum PON1 activity was assessed enzymatically (NW *n*=44, obese *n*=97); and wherein the obese cohort was subdivided into MHO (*n*=43) and MUO (*n*=65) and (E) total, (F) ABCA1 -independent and (G) ABCA1-dependent efflux presented; and wherein (H) Serum PON1 activity within the MHO (*n*=35) and MUO (*n*=58) sub-groups are presented; wherein statistical significance is presented as *p<0.05, **p<0.01, ***p<0.001 w.r.t. NW;

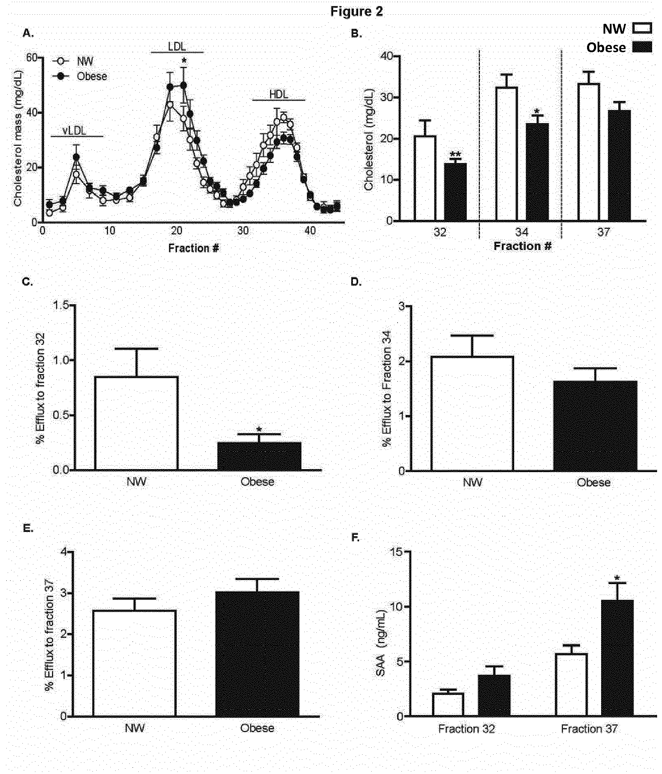

**Figure 2** illustrates fractionation and assessment of HDL sub-particle function; wherein a sub-group of age and sex-matched NW (*n*=12) and obese (*n*=17) subjects were selected and serum separated by FPLC; wherein (A) Cholesterol levels within fractions were determined enzymatically and a lipoprotein profile curve generated; and wherein (B) Cholesterol levels within fractions 32, 34 and 37 presented; and wherein J774 macrophages were labeled with ³H-cholesterol (1µCi/ml), and stimulated with cAMP (0.3mM) prior to evaluation of efflux to 30% v/v FPLC fraction over 4h; and wherein Total efflux to (C) HDL-fraction 32 (large), (D) HDL-fraction 34 (medium) and (E) HDL-fraction 37 (small) are presented; and wherein (F) SAA protein levels were determined within fraction 32 and 37 by ELISA; wherein statistical significance is presented as *p<0.05, **p<0.01, ***p<0.001 w.r.t. NW;

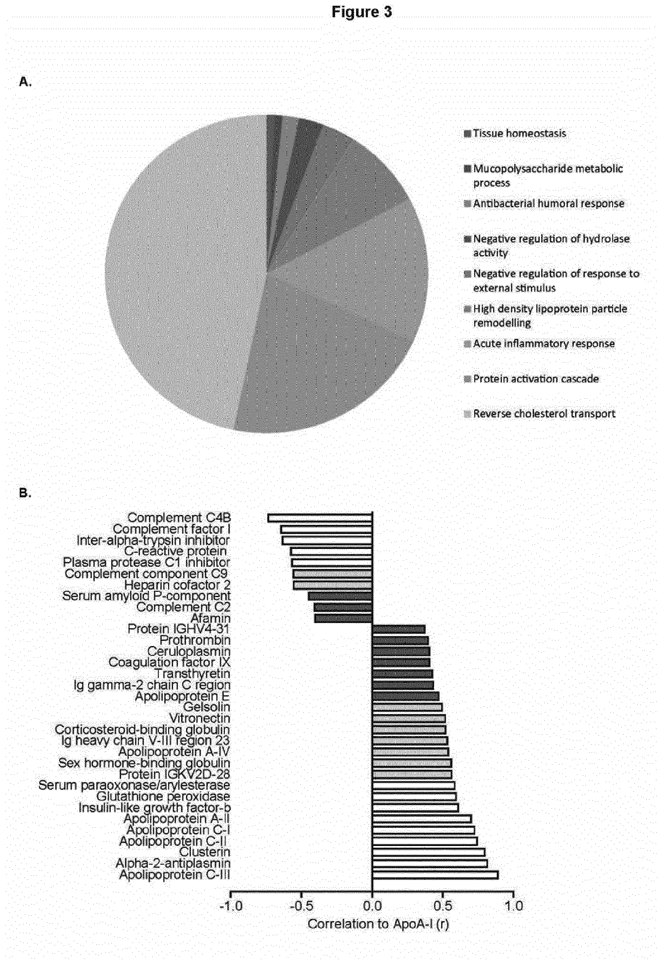

**Figure** 3 illustrates functional analysis of the HDL proteome; wherein serum lipoproteins (NW n=12, Obese n=17 (MHO *n*=7; MUO *n*=10), *n*=29 total) were separated by FPLC and protein within fraction 38 was precipitated, digested and proteomics characterised using an Orbitrap Q Exactive mass spectrometer; and wherein raw data was processed using MaxQuant and analysed using Perseus software; wherein gene ontology (GO) analysis was performed on the data from the total cohort (*n*=29); and wherein (A) Statistically enriched categories (p<0.05) were functionally grouped and visualised using a pie chart; and wherein the most significant terms in each functional group are labelled; and wherein (B) Non parametric correlations were performed between ApoA-I and the HDL proteome across the total cohort, and significant correlations are presented;

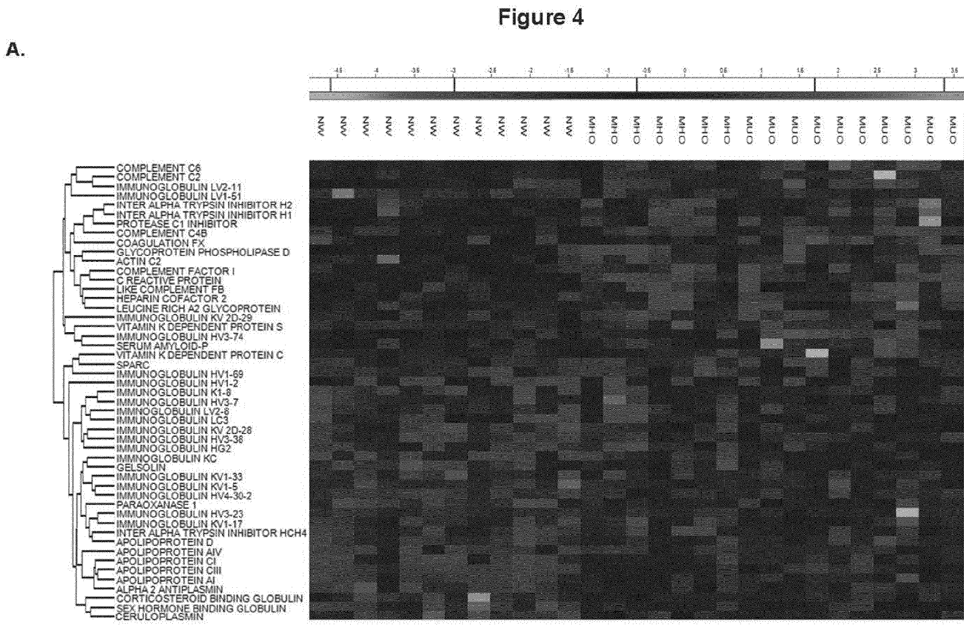

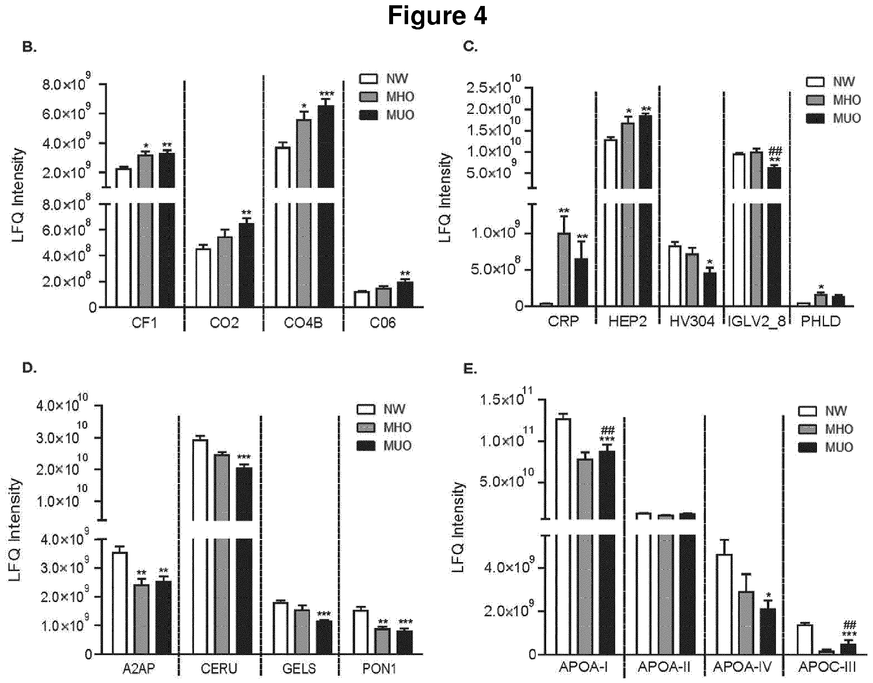

**Figure 4** illustrates visualisation of differentially regulated proteins on HDL-fraction 38 between NW and MUO groups; wherein (A) the differentially regulated proteins (p<0.05) on HDL fraction 38 comparing LFQ intensities between NW (n=12) and MUO (*n*=10) were visualised using a heat map in Perseus; wherein, for visualisation, Log2 transformed LFQ intensities are first imputed to replace missing values with values from a normal distribution and subsequently z-score normalized; wherein label free quantitative (LFQ) intensities of (B) CF1, CO2, CO4B and C06, (C) CRP, HEP2, HV304, IGLV2_8 and PHLD, (D) A2AP, CERU, GELS and PON1 and (E) APOA-I, APOA-II, APOA-IV and APOC-III are illustrated (*p<0.05, "p<0.01, ***p<0.001 w.r.t. NW; ##p<0.01 w.r.t. MHO);

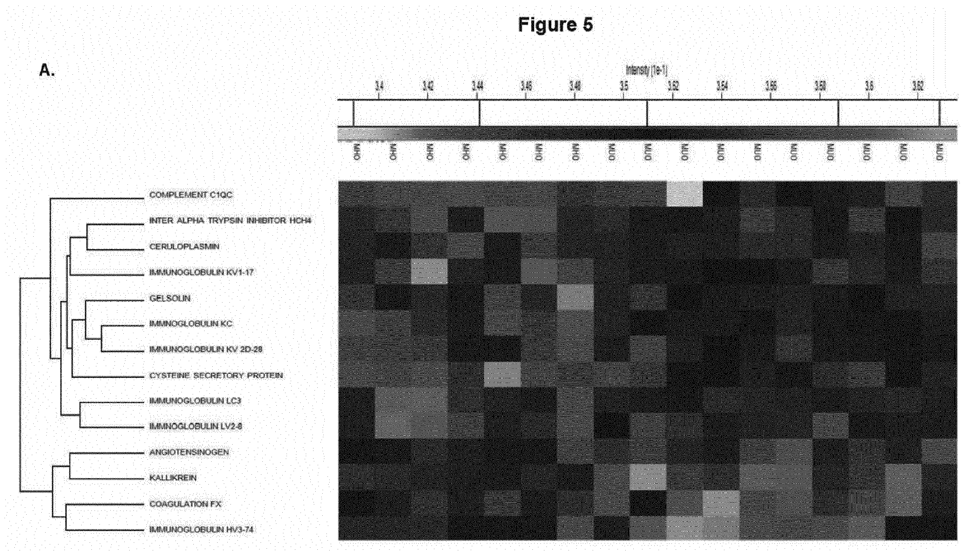

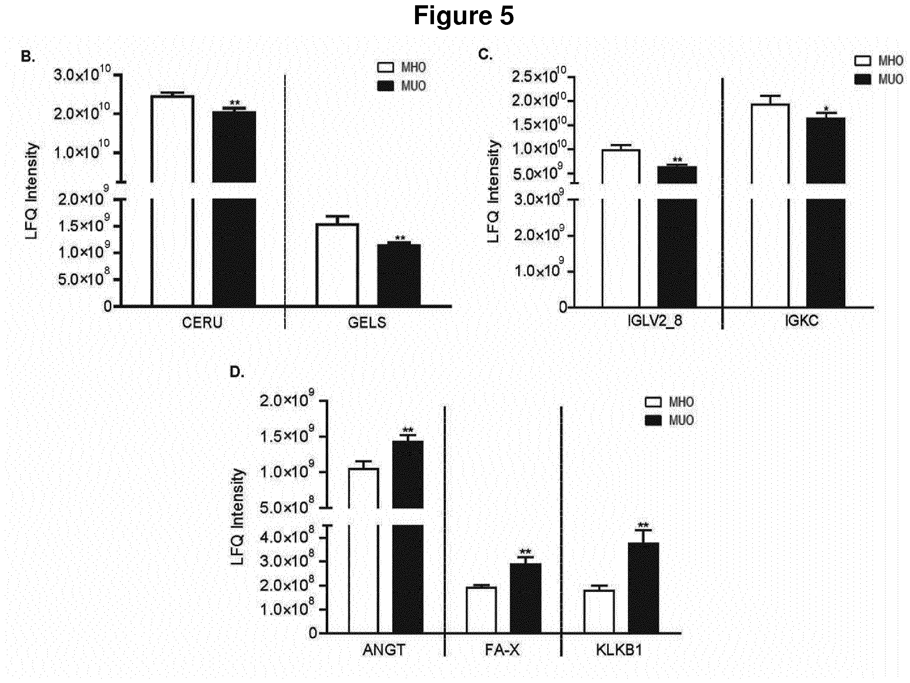

**Figure 5** illustrates visualisation of differentially regulated proteins on HDL between MHO and MUO groups; wherein (A) the differentially regulated proteins (p<0.05) on HDL fraction 38 comparing LFQ intensities between MHO (*n*=7) and MUO (*n*=10) were visualised using a heat map in Perseus; wherein, for visualisation, Log2 transformed LFQ intensities are first imputed to replace missing values with values from a normal distribution and subsequently z-score normalized; wherein label free quantitative (LFQ) intensities of (B) CERU and GELS, (C) IGLV2_8 and IGKC and (D) ANGT, FA-X and KLKB1 were illustrated, (*p<0.05, "p<0.01, w.r.t. NW);

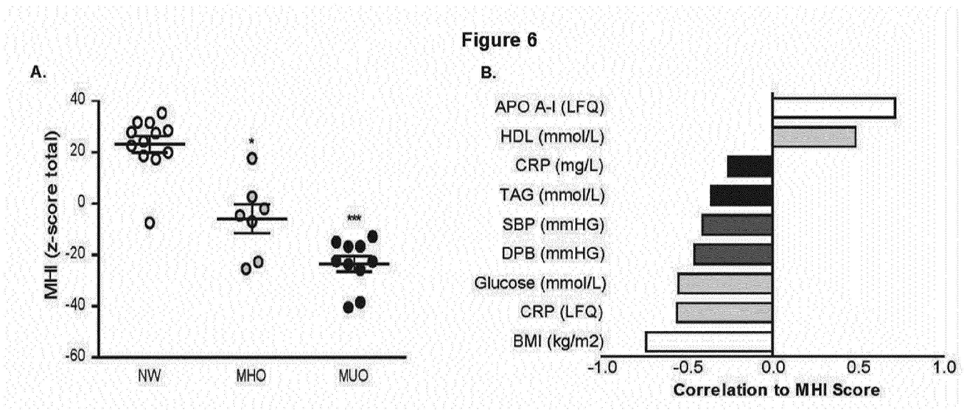

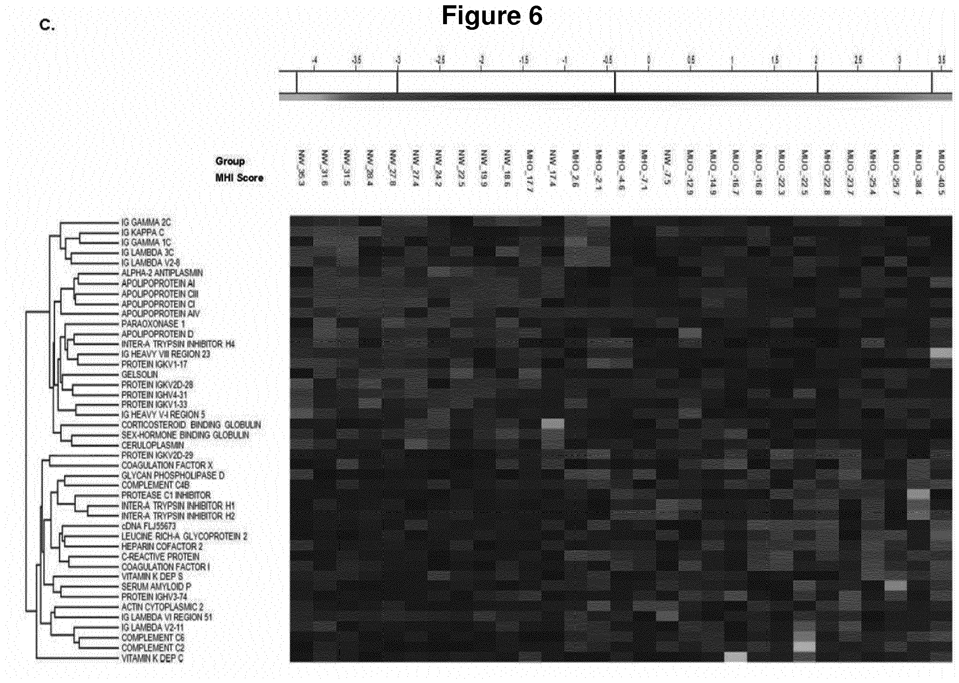

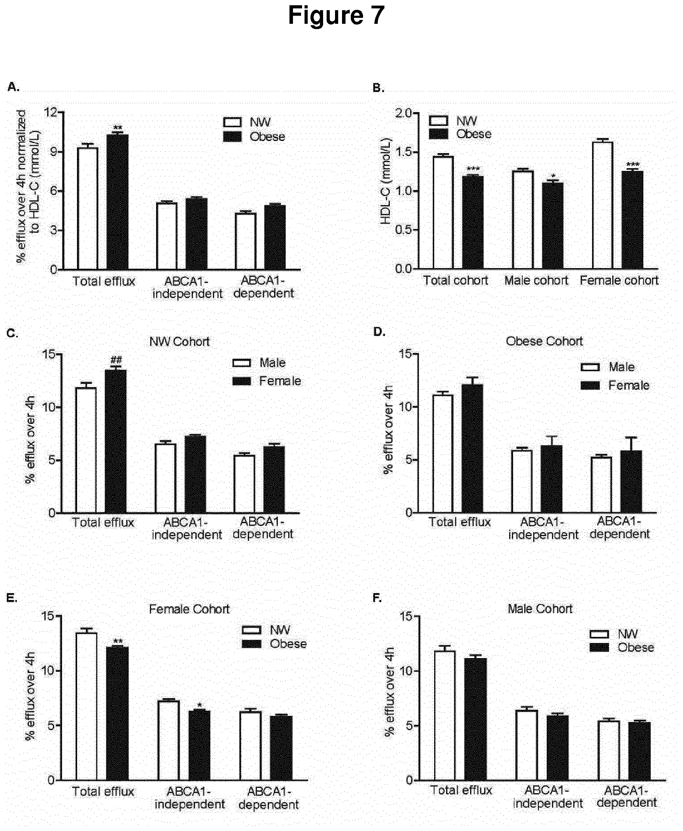

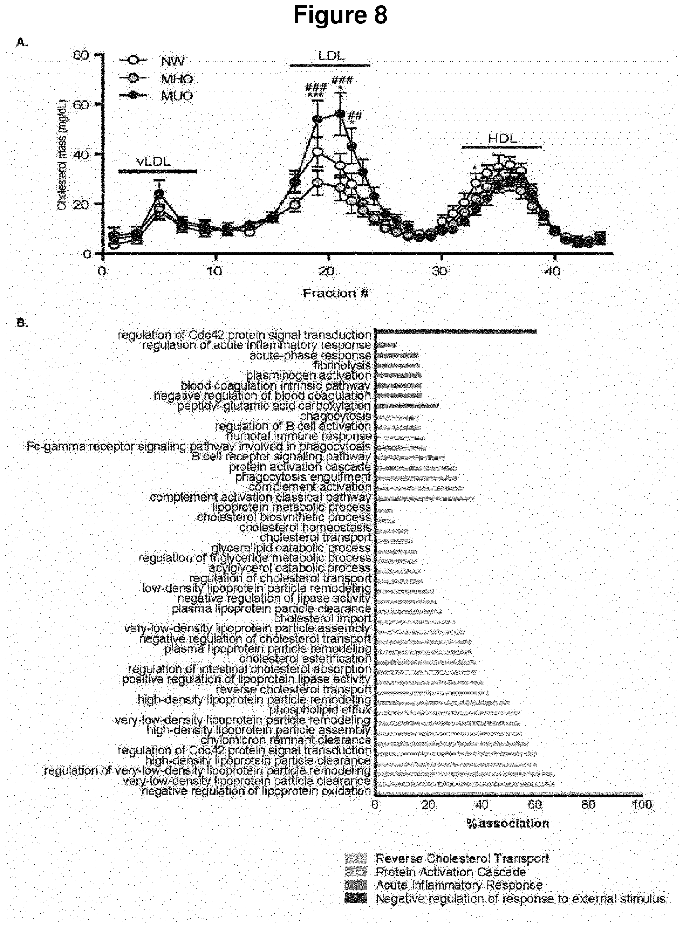

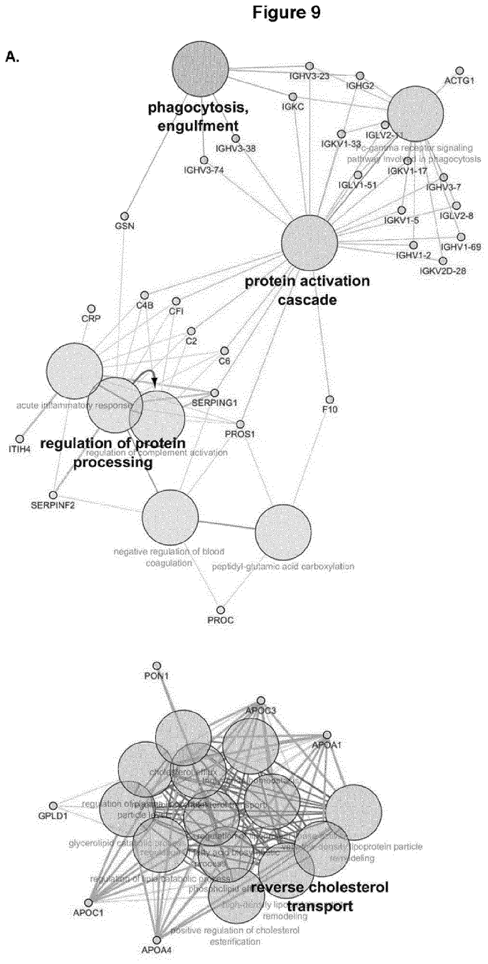

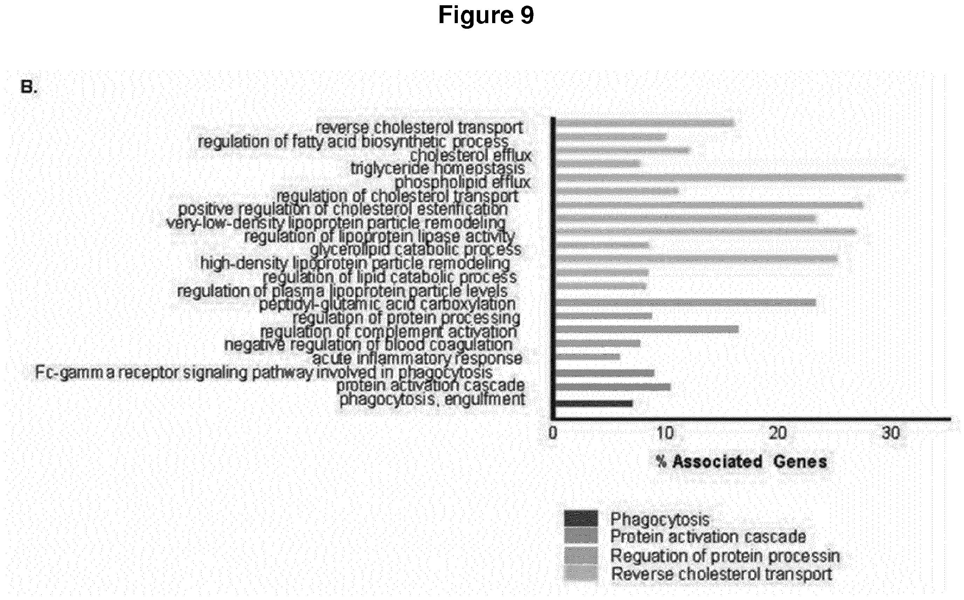

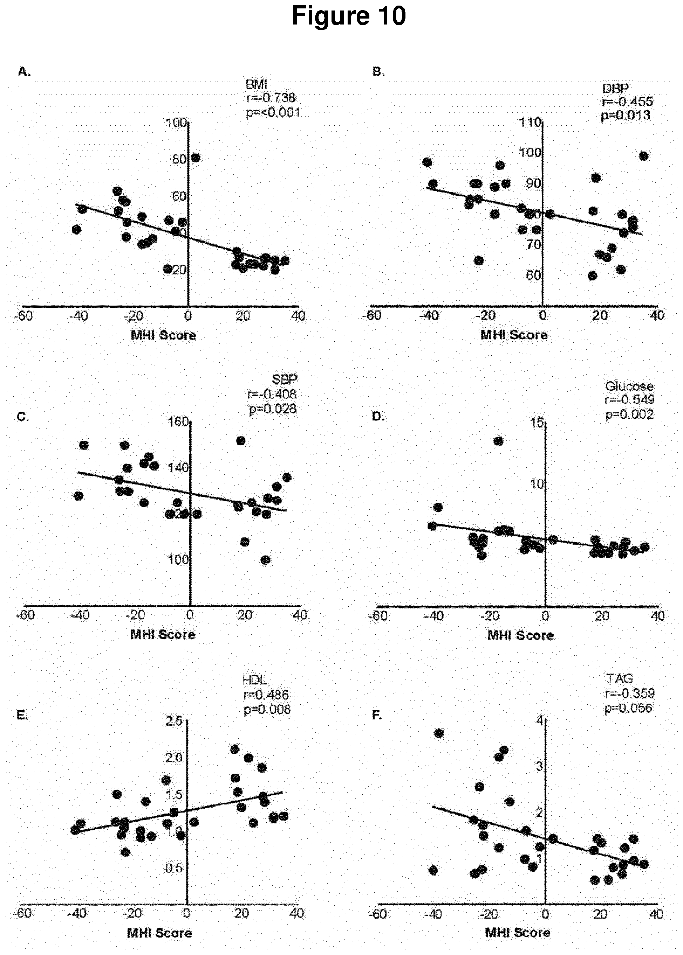

**Figure** 6 illustrates metabolic HDL index score - correlation to metabolic health parameters; wherein (A) a Metabolic HDL Index (MHI) score was developed using the 44 significantly different proteins between NW and MUO groups and the composite score across groups is presented, *p<0.05 and ***p<0.001 ; wherein (B) non parametric correlations were performed between the MHI score and clinical characteristics; and wherein (C) a heat-map was generated in Perseus based on the contributing proteins to the MHI score and were ranked according to decreasing MHI score;

**Supplement** **Figure 1** illustrates cholesterol efflux capacity normalised to HDL-C and characterisation of gender effects on CEC within NW and obese cohort J774 macrophages, labeled with 3H-cholesterol (1µCi/ml), were stimulated ± cAMP (0.3mM) to drive ABCA1 protein expression; wherein fasting serum samples were depleted of ApoB particles by PEG precipitation; and wherein serum HDL-C levels were determined enzymatically; and wherein percentage ³Hcholesterol efflux to 2.8% PEG-supernatant was monitored over 4h and normalised to HDLC input; wherein (A) Total, ABCA1-independent and ABCA1-dependent efflux capacity after normalisation to HDL-C; (B) Serum HDL-C levels within NW (n=129) and obese (n=1 10) cohort and male (NW n =129, Obese n=110) and female (NW n=129, Obese n=110) subgroups; and wherein non-normalised efflux in male and female (C) NW and (D) obese cohorts presented; and wherein effects of obesity on efflux in (E) female and (F) male cohorts presented compared to NW; wherein statistical significance is presented as *p<0.05, **p<0.01, ***p<0.001 w.r.t. NW, ##p<0.01, male v female;

**Supplement** **Figure 2** illustrates functional analysis of the differentially expressed proteins on the HDL proteome; wherein a sub-group of age and sex-matched NW (n=12) and Obese n=17 (MHO n=7; MUO n=10) subjects were selected and serum separated by FPLC (A) 146 proteins were identified; and wherein enriched categories from GO biological processes analysis of the genes were functionally grouped using GO Term Fusion; and wherein the percentage genes associated with the functional pathways were identified using GO analysis and presented in bar graph format (B);

**Supplement** **Figure 3** illustrates functional analysis of the differentially expressed proteins on the HDL proteome between NW and MUO groups; wherein a sub-group of age and sex-matched NW (n=12) and Obese n=17 (MHO n=7; MUO n=10) subjects were selected and serum separated by FPLC; wherein the functional pathways associated with the differentially expressed proteins on HDL between NW and MUO groups were investigated using GO analysis (A); and wherein enriched categories from GO biological processes analysis of the genes were functionally grouped using GO Term Fusion; wherein the primary pathways identified by GO analysis are presented in bar-graph format (B); and

**Supplement** **Figure 4** illustrates correlation between MHI score and components of the metabolic Syndrome; wherein non-parametric correlations were applied to assess the relationship between the MHI score and components of the Metabolic Syndrome; BMI (kg/m2) (A), DBP (mmHG) (B), SBP (mmHG) (C), Glucose (mmol/L) (D), HDL (mmol/L) (E) and TAG (mmol/L) (F).

### Examples

Reference will now be made to the following non-limiting examples:

### Materials and Methods

**Materials:** Cholesterol [1,2-3H(N)] was purchased from Perkin-Elmer Analytical Sciences (Ireland). Cell culture material was purchased from Lonza (Slough, UK). All other reagents, unless otherwise stated, were from Sigma Aldrich Ltd.

**Study Population:** The study subjects (n=108 obese and n=131 normal weight (NW)) were recruited by St. Vincent's University Hospital, University College Dublin and Tallaght Hospital, Dublin, Ireland. Overnight fasted serum samples were used for all analysis. The inclusion criteria for the obese subjects were: age (20 to 70 years), BMI ≥ 30kg/m², while the inclusion criteria for the normal weight (NW) control subjects were: age (20 to 70 years old), BMI < 30kg/m², and absence of the MetS (MetS). Obese subjects were classified into metabolically healthy obese (MHO, n=43) (≤2 components of MetS) or metabolically unhealthy obese (MUO, n=65) groups (≥3 components of MetS) based on the following National Cholesterol Education Program - Adult Treatment Panel III (NCEP-ATP III) guidelines [3]; (1) waist circumference >102cm (men) and >88cm (women) (2) Triglycerides levels ≥ 150 mg/dL, (3) HDL-C levels < 40 mg/dL (men) and < 50 mg/dL (women) (4) fasting glucose levels ≥ 100 mg/dL and (5) systolic blood pressure ≥130 mmHg and diastolic blood pressure ≥ 85 mmHg. A sub-group of age and sex-matched NW (*n*=12) and obese (*n*=17; *n*=7 MHO & *n*=10 MUO) were selected for HDL proteomics analysis. Ethical approval was obtained from University College Dublin, St. Vincent's University Hospital and Tallaght Hospital Human Research Ethics committees.

**Paraoxonase-1 (PON1) Activity Assay:** PON1 activity was determined by the conversion rate of phenylacetate to phenol in the presence of serum as described in Osto, E., et al., 2015. 131(10): p. 871-81.25. Activity of PON-1 is expressed as per µmol/min/L.

**Fast Protein Liquid Chromatography (FPLC):** Lipoproteins from frozen serum samples (150µL) were separated using size exclusion chromatography on FPLC (Amersham Pharmacia Biotech) using two sequential Superose 6 10/300 columns (GE Healthcare Lifesciences, UK) and phosphate buffer saline containing 1mM Ethylenediaminetetraacetic acid as the eluent. Cholesterol concentration in each fraction was determined enzymatically using LabAssay^{™} Cholesterol (WAKO chemicals, Germany). FPLC fractions were stored at -80°C prior to proteomics analysis.

**Cholesterol efflux capacity (CEC):** J774 macrophages were labeled for 24h with ³H-cholesterol (1µCi/ml) and equilibrated overnight in Dulbecco's modified eagle medium (DMEM) containing 0.2% bovine serum albumin (BSA) ± cAMP (0.3mM) to drive ABCA1 expression. ApoB-containing lipoproteins were removed from human serum by polyethylene glycol (PEG) precipitation as described in Vikari, J., 1976. 36(3): p. 265-8 or HDL-fractions were isolated by FPLC. *Ex vivo* efflux from labeled macrophages to 2.8% HDL supernatant or 30% v/v FPLC fraction in minimal essential media (MEM) was measured over 4h. The difference in efflux from cells stimulated in the presence or absence of cAMP represents ABCA1-dependent efflux. ABCA1-independent efflux was derived from untreated (-cAMP) cells.

**Proteomics analysis:** Lipoproteins from serum samples were separated by FPLC and proteins from HDL-containing fraction 38 were precipitated using trichloroacetic acid (TCA). Protein pellets washed with ice-cold acetone and re-suspended in buffer of 8M Urea in 50mM Ammonium Bicarbonate (NH₄HCO₃, Sigma Aldrich). Protein concentration was determined using Bradford Protein Assay. Cysteines of plasma protein samples were reduced using dithiothreitol followed by alkylation with iodoacetamide before addition of trypsin (trypsin singles TM proteomic grade, Sigma Aldrich). Digestion was carried out overnight at 37°C. After drying in vacuum centrifuge, peptides were acidified by trifluoroacetic acid (TFA), desalted with c18 STAGE tips as described in Rappsilber, J., 2007. 2(8): p. 1896-906 and re-suspended in 0.1% TFA. The samples were run on a Thermo Scientific Q Exactive mass spectrometer connected to a Dionex Ultimate 3000 (RSLC nano) chromatography system. Raw data was processed using MaxQuant version 1.5.5.1 incorporating the Andromeda search engine. MS/MS spectra was searched against a human uniprot database using the default settings of MaxQuant. Label free quantitative (LFQ) ion intensities of peptides and proteins were generated by MaxQuant and analysed using Perseus software. Data was log transformed and t-test comparison of fractions carried out. For visualization using heat maps, missing values were imputed with values from a normal distribution and the dataset was normalized by z-score as decrbied by Tyanova, S., et al., 2016. 13(9): p. 731-40.

**Serum analysis:** Serum insulin was measured by ELISA (Crystal Chem Inc, USA). Plasma and PEG-supernatant triacylglycerol (TAG), cholesterol, phospholipid (Wako Chemicals GmbH, Germany), were measured enzymatically as per manufacturers' guidelines.

**Statistical analysis and metabolic HDL index (MHI) scoring:** Statistical analysis was performed using GraphPad Prism 5 (GraphPad Sofware Inc, CA) and SPSS software (IBM analytics). Normality tests were conducted using Shapiro-Wilk tests prior to analysis. In the event of normally distributed data, a one-way ANOVA with Bonferroni post-hoc test was applied to data-sets with multiple groups, and an unpaired t-test applied to data-sets of two groups. An adjusted General Linear Model was used to assess confounding. If violation of normal distribution was observed, non-parametric Kruskal-Wallis with a Dunn's post-hoc test was applied to data-sets with multiple groups. Bivariate correlations were performed using Pearson's (normal data) or Spearman's (non-normal data) tests as appropriate. Variables are expressed as mean ± SEM. To generate a MHI score from the proteomics data-base, z-scores were generated from raw LFQ values and the sum of the z-scores of proteins that increased in MUO was subtracted from the sum of the z-scores that decreased in MUO.

### Example 1

### Serum ABCA1-independent efflux capacity and PON1 activity reduced in obese subjects compared to normal-weight (NW) control

Clinical characteristics of NW and obese cohorts are highlighted in Table 1 and the medication list is provided within the supplement (Supplement Table 1A&B). The obese group exhibited significant increases in BMI and waist to hip ratio, triglyceride (TAG), fasting glucose, hsCRP, fasting insulin, and HOMA-IR while HDL-C was significantly reduced compared to NW. A significant reduction in total and ABCA1-independent efflux to ApoB-depleted serum was observed in the obese group compared to NW group. No effect on ABCA1-dependent efflux was observed (Figure 1A-C). Reduced HDL-C was a critical determinant of reduced efflux as evident upon normalisation of results to HDL-C (Supplement Figure 1A&B). Total and ABCA1-independent efflux negatively correlated with BMI, systolic BP, diastolic BP, TAG and insulin and positively correlated with HDL-C across the total cohort (Table 2A/B). ABCA1-dependent efflux positively correlated with HbA1c (r=0.212) while ABCA1-independent efflux negatively correlated with HbA1c (r= -0.214) indicative of a shift from efflux to larger particles towards smaller particles with insulin resistance. A significant correlation between efflux and gender was also observed (Table 2A/B) with NW females exhibiting significantly higher CEC compared to NW males - this beneficial effect was abrogated within the obese setting (Supplement Figure 1C&D). Furthermore the reduction in efflux within the obese cohort compared to lean cohort was more pronounced in females than in males (Supplement Figure 1E&F). These findings suggest that the protective effect of female gender is negated in the obese setting.

**Table 1. Participant Characteristics**

| | **Lean** | **Obese** | |
|---|---|---|---|
| | *n*=131 | *n*=108 | |
| | Mean ± SEM | Mean ± SEM | **P*** |
| Age (years) | 40.23 ± 0.82 | 45.10 ± 1.14 | **<0.001** |
| Gender (M,F %) † | 49, 51 | 42, 58 | 0.267 |
| BMI (kg/m²) | 25.05 ± 0.24 | 45.77 ± 0.97 | **<0.001** |
| Waist to Hip Ratio | 0.85 ± 0.01 | 0.96 ± 0.02 | **0.010** |
| Systolic Blood Pressure (mmHG) | 121.68 ± 2.07 | 131.53 ± 1.25 | 0.113 |
| Diastolic Blood Pressure (mmHG) | 74.70 ± 1.34 | 82.40 ± 0.87 | 0.053 |
| Total Cholesterol (mmol/L) | 4.67 ± 0.08 | 4.73 ± 0.09 | 0.241 |
| HDL (mmol/L) | 1.45 ± 0.03 | 1.19 ± 0.03 | **0.003** |
| LDL (mmol/L) | 2.65 ± 0.12 | 2.69 ± 0.10 | 0.538 |
| TAG (mmol/L) | 0.99 ± 0.04 | 1.58 ± 0.08 | **0.001** |
| HbA1c (mmol/mol) | 32.64 ± 1.22 | 41.43 ± 1.12 | 0.253 |
| Glucose (mmol/L) | 5.17 ± 0.06 | 5.91 ± 0.25 | **<0.001** |
| Insulin (mIU/L) ‡ | 6.50 ± 0.56 | 24.03 v 2.10 | **<0.001** |
| HOMA-IR ‡ | 1.54 ± 0.15 | 6.45 ± 0.83 | **<0.001** |
| High sensitivity CRP (mg/L) § | 1.63 ± 0.31 | 7.90 ± 0.76 | **<0.001** |

| | | | |
|---|---|---|---|
| *Differences assessed by Independent Samples T-Test † Differences assessed by a X² Test ‡ n=68 (NW), 53 (Obese) § n= 117 (NW), 98 (Obese) | | | |

**Table 2A. Correlations between Efflux Capacity and Clinical Characteristics**

| | **Total Efflux** | | | | | |
|---|---|---|---|---|---|---|
| | Total Cohort | | Lean Cohort | | Obese Cohort | |
| | *n*=239 | | *n*=129 | | *n*=110 | |
| | **R** | **P** | **R** | **P** | **R** | **P** |
| Age (years) | 0.022 | 0.731 | 0.188 | 0.031 | -0.082 | 0.402 |
| Gender (M%,F%) | **0.227** | **<0.001** | **0.241** | **0.006** | **0.258** | **0.007** |
| BMI (kg/m²) | **-0.157** | **0.015** | -0.111 | 0.207 | 0.034 | 0.730 |
| SBP (mmHG) | **-0.204** | **0.002** | -0.221 | 0.012 | -0.101 | 0.298 |
| DBP (mmHG) | **-0.198** | **0.002** | **-0.190** | **0.031** | -0.097 | 0.317 |
| TC(mmol/L) | -0.032 | 0.626 | -0.046 | 0.604 | 0.059 | 0.548 |
| HDL (mmol/L) | **0.286** | **<0.001** | **0.202** | **0.020** | **0.327** | **0.001** |
| LDL (mmol/L) | -0.105 | 0.208 | -0.183 | 0.190 | -0.005 | 0.961 |
| TAG (mmol/L) | **-0.147** | **0.023** | -0.069 | 0.433 | -0.066 | 0.501 |
| Glucose (mmol/L) | -0.030 | 0.658 | -0.086 | 0.360 | 0.135 | 0.166 |
| Insulin (uIU/ml) | **-0.235** | **0.009** | -0.009 | 0.942 | -0.042 | 0.769 |
| HOMA-IR | -0.165 | 0.069 | -0.036 | 0.767 | 0.052 | 0.710 |
| HbA1 c(mmol/mol) | -0.018 | 0.834 | -0.002 | 0.987 | -0.046 | 0.678 |
| hsCRP (mg/L) | -0.127 | 0.064 | **-0.184** | **0.045** | 0.012 | 0.908 |

| | | | | | | |
|---|---|---|---|---|---|---|
| Spearman Correlations | | | | | | |

**Table 2B. Correlations between Efflux Capacity and Clinical Characteristics**

| | **ABCA1-Independent Efflux** | | | | | |
|---|---|---|---|---|---|---|
| | Total Cohort | | Lean Cohort | | Obese Cohort | |
| | *n*=239 | | *n*=131 | | *n*=108 | |
| | **R** | **P** | **R** | **P** | **R** | **P** |
| Age (years) | 0.070 | 0.280 | 0.194 | 0.026 | 0.072 | 0.456 |
| Gender (M%,F%) | **0.243** | **<0.001** | **0.339** | **<0.001** | 0.142 | 0.143 |
| BMI (kg/m²) | **-0.175** | **0.007** | -0.069 | 0.437 | -0.035 | 0.718 |
| SBP (mmHG) | **-0.190** | **0.003** | **-0.231** | **0.008** | -0.021 | 0.827 |
| DBP(mmHG) | **-0.173** | **0.007** | -0.143 | 0.107 | -0.059 | 0.546 |
| TC(mmol/L) | -0.097 | 0.137 | -0.136 | 0.122 | -0.006 | 0.952 |
| HDL (mmol/L) | **0.308** | **<0.001** | **0.276** | **0.001** | **0.245** | **0.012** |
| LDL (mmol/L) | **-0.208** | **0.012** | **-0.429** | **0.001** | -0.067 | 0.525 |
| TAG (mmol/L) | **-0.172** | **0.008** | -0.077 | 0.385 | -0.122 | 0.212 |
| Glucose (mmol/L) | -0.075 | 0.266 | -0.177 | 0.059 | 0.092 | 0.346 |
| Insulin (uIU/ml) | -0.179 | 0.050 | 0.125 | 0.303 | -0.225 | 0.113 |
| HOMA-IR | -0.143 | 0.114 | 0.086 | 0.478 | -0.141 | 0.314 |
| HbA1 c(mmol/mol) | **-0.214** | **0.012** | **-0.338** | **0.013** | -0.031 | 0.781 |
| hsCRP (mg/L) | **-0.141** | **0.039** | -0.135 | 0.143 | -0.075 | 0.468 |

| | | | | | | |
|---|---|---|---|---|---|---|
| Spearman Correlations | | | | | | |

**Supplement Table 1A. Medication use in the obese cohort, split by MHO and MUO**

| | **MHO** | **MUO** | **P*** |
|---|---|---|---|
| | *n*=43 | *n*=65 | |
| Medication Use | 79.9 | 84.3 | 0.268 |

| **Medication Type** | | | |
|---|---|---|---|
| Insulin-sensitising | 15.4 | 35.3 | **0.029** |
| Weight loss | 25.6 | 25.5 | 0.588 |
| Liraglitide | 12.8 | 29.4 | 0.051 |
| Statins | 10.3 | 17.6 | 0.249 |
| Hypertension | 23.1 | 39.2 | 0.081 |

| | | | |
|---|---|---|---|
| * Differences between groups assessed using X2 Test | | | |

**Supplement Table 1B. Medication Types**

| Insulin-sensitising | Weight Loss | Liraglitide | Statins | Anti-hypertensives |
|---|---|---|---|---|
| Diaglyc | Orlistat | Victoza | Atorvastatin | Accupo |
| Diamicron | Reductil | | Lipitor | ACE-1 |
| DPP4 | Subitramine | | Provastatin | Amlode |
| Glicazide | Xenical | | Simvastatin | Amlodipine |
| Glucophage | | | | Atenelol |
| Junamet | | | | Bendroflumethiazide |
| Metformin | | | | Benetor |
| Novarapid | | | | Bisoprolol |
| Trajenta | | | | Biopress |
| | | | | Cardura XL |
| | | | | Centyl K |
| | | | | Cosartal |
| | | | | Coversil |
| | | | | Cozaar |
| | | | | Doxatosin |
| | | | | Frusemide |
| | | | | Half-beta progone |
| | | | | Istin |
| | | | | Konverge |
| | | | | Lercanidipine |
| | | | | Lisinopril |
| | | | | Losartan |
| | | | | Metaprolol |
| | | | | Metocor |
| | | | | Micardis plus |
| | | | | Micordis |
| | | | | Modiuretic |
| | | | | Nebivolol |
| | | | | Omessar Plus |
| | | | | Perindopril |
| | | | | Ramic |
| | | | | Rampiril |
| | | | | Telmisartan |
| | | | | Zestril |

Serum PON1 activity was significantly reduced in the obese group relative to NW (Figure 1D). The obese group was sub-divided into MHO and MUO sub-groups and clinical characteristics outlined in Table 3. A significant difference in HDL-C, TAG, fasting glucose and HbA1c was observed between MHO and MUO. Both groups exhibited reduced ABCA1 -independent efflux and PON-1 activity compared to NW with no significant difference evident between MHO and MUO (Figure 1E-H).

**Table 3. Participant characteristics, split by MHO and MUO**

| | **NW** | **MHO** | **MUO** | | |
|---|---|---|---|---|---|
| | *n*=131 | *n=43* | *n=65* | | |
| | Mean ± SEM | Mean ± SEM | Mean ± SEM | P* | P† |
| Age (years) | 40.05 ± 0.83^{a} | 43.49 ± 1.73^{ab} | 46.42 ± 1.48^{b} | **<0.001** | |
| Gender (M,F %) ‡ | 50,50 | 36, 64 | 45,55 | 0.260 | |
| BMI (kg/m²) | 25.06 ± 0.24^{a} | 44.34 ± 1.72^{b} | 46.11 ± 1.18^{b} | **<0.001** | **<0.001** |
| Waist to Hip Ratio | 0.85 ± 0.01^{a} | 0.92 ± 0.02^{b} | 0.98 ± 0.03^{b} | **<0.001** | **<0.001** |
| SBP (mmHG) | 121.61 ± 2.09^{a} | 126.91 ± 2.21^{ab} | 134.55 ± 1.41^{b} | **<0.001** | **0.002** |
| DBP(mmHG) | 74.67 ± 1.35^{a} | 81.02 ± 1.37^{b} | 83.18 ± 1.15^{b} | **<0.001** | **<0.001** |
| Total Cholesterol (mmol/L) | 4.67 ± 0.08 | 4.70 ± 0.11 | 4.74 ± 0.12 | 0.902 | 0.978 |
| HDL (mmol/L) | 1.44 ± 0.03^{a} | 1.33 ± 0.05^{a} | 1.12 ± 0.03^{b} | **<0.001** | **<0.001** |
| LDL (mmol/L) | 2.67 ± 0.12 | 2.68 ± 0.14 | 2.67 ± 0.15 | 0.997 | 0.927 |
| TAG (mmol/L) | 0.99 ± 0.04^{a} | 1.14 ± 0.04^{a} | 1.87 ± 0.12^{b} | **<0.001** | **<0.001** |
| HbA1c (mmol/mol) § | 34.79 ± 3.41^{a} | 38.25 ± 1.23^{b} | 43.35 ± 1.71^{c} | **<0.001** | **<0.001** |
| Glucose (mmol/L) | 5.18 ± 0.06^{a} | 4.99 ± 0.24^{a} | 6.52 ± 0.37^{b} | **<0.001** | **<0.001** |
| Insulin (uIU/ml) § | 6.27 ± 0.52^{a} | 22.54 ± 3.08^{b} | 24.34 ± 2.75^{b} | **<0.001** | **<0.001** |
| HOMA-IR § | 1.49 ± 0.14^{a} | 4.96 ± 1.00^{b} | 7.11 ± 1.11^{b} | **<0.001** | **<0.001** |
| High sensitivity CRP (mg/L) II | 1.65 ± 0.31^{a} | 6.96 ± 1.12^{b} | 8.29 ± 1.11^{b} | **<0.001** | **<0.001** |

| | | | | | |
|---|---|---|---|---|---|
| * Differences across groups were assessed using a one-way ANOVA with Bonferroni Correction † Differences across groups were assessed using a General Linear Model adjusted for Age ^{a}'^{b}'^{c} Mean values with unlike superscript letters are significantly different between groups (P<0.05) † Differences across groups were assessed using a *X²* Test § *n*=68 (NW), 20 (MHO), 33 (MUO) ∥ *n*= 117 (NW), 40 (MHO), 58 (MUO) | | | | | |

### Example 2

### Lipoprotein separation and sub-particle functionality

A sub-cohort of age and sex-matched individuals were selected from NW (n=12) and obese (n=17) groups (Table 4A) and serum lipoproteins were separated by FPLC (Figure 2A). Cholesterol on LDL fraction 21 was significantly increased in the obese group compared to NW, with a trend towards reduced cholesterol on larger HDL fractions (fractions 30-36), with no difference in cholesterol levels on smaller HDL fractions (Figure 2A). We subsequently assessed the efflux capacity of FPLC fractions containing large (fraction 32), medium (fraction 34) and small (fraction 37) HDL particles. Cholesterol concentration was reduced on fractions 32&34, with no difference in fraction 37 (Figure 2B), in obese group compared to NW. The efflux function of larger HDL particles was significantly reduced (Figure 2C), with no difference in efflux to medium and small HDL fractions (Figures 2D&E). SAA levels were significantly increased on obese-HDL fraction 37 as detected by ELISA compared to NW (Figure 2F).

**Table 4A. Characteristics of age and sex matched sub-cohort**

| | **NW** | **Obese** | |
|---|---|---|---|
| | *n*=12 | *n*=17 | P* |
| Age (years) | 41.25 ± 2.53 | 41.76 ± 2.16 | 0.879 |
| Gender (M, F %) † | 50,50 | 53, 47 | 0.587 |
| BMI (kg/m²) | 23.64 ± 0.68 | 47.60 ± 3.05 | **<0.001** |
| SBP (mmHG) | 124.25 ± 3.78 | 132.59 ± 2.50 | 0.065 |
| DBP (mmHG) | 75.42 ± 3.40 | 84.18 ± 1.98 | **0.028** |
| TAG (mmol/L) | 1.02 ± 0.09 | 1.72 ± 0.24 | **0.013** |
| Cholesterol (mmol/L) | 4.43 ± 0.19 | 4.75 ± 0.26 | 0.366 |
| HDL (mmol/L) | 1.50 ± 0.10 | 1.11 ± 0.06 | **0.001** |
| LDL (mmol/L) | 2.46 ± 0.24 | 2.87 ± 0.20 | 0.189 |
| HbA1c (mmol/mol) | 29.34 ± 1.69 | 44.70 ± 5.21 | **0.023** |
| Glucose (mmol/L) | 4.70 ± 0.09 | 6.12 ± 0.51 | **0.028** |
| Insulin (mmol/L) † | 7.38 ± 3.18 | 29.33 ± 4.15 | **0.001** |
| HOMA-IR † | 1.43 ± 1.41 | 8.23 ± 1.50 | **0.001** |
| High sensitivity CRP (mg/L) ‡ | 0.65 ± 0.10 | 9.00 ± 2.23 | **0.003** |

| | | | |
|---|---|---|---|
| n, number of participants. * Differences across groups were assessed using an Independent Samples T-Test † n=6 (NW), n=15 (Obese), ‡ n=9 (NW), n=12 (Obese) | | | |

### Example 3

### HDL proteomic profile modulated in obesity

The obese group was sub-divided into MHO (n=7) and MUO (n=10) sub-groups (clinical parameters outlined in Table 4B) and proteomics was carried on HDL fraction 38 to determine whether proteins pertaining to other HDL functions, beyond efflux capacity, were altered dependent upon metabolic health status. HDL proteomics was performed on FPLC fraction 38 where no significant difference in HDL-C was evident across groups (Supplement Figure 2A). 146 proteins were identified on the HDL particles and gene ontology (GO) analysis was performed within Cytoscape across the combined cohort. The primary pathways identified included high density lipoprotein particle remodelling, acute inflammatory response, protein activation cascade and reverse cholesterol transport (Figure 3A and Supplement Figure 2B). Given ApoA-I is the primary protein on HDL we correlated levels of ApoA-I with the proteomic data-set across the combined cohort (Figure 3B). ApoA-I positively correlated with ApoC-III, Alpha-2-antitrypsin, clusterin, ApoC-II, ApoC-I and ApoA-II and negatively correlated with complement factor 4B (CO4B), complement factor 1 (CF1) inter-alpha-trypsin inhibitor and C-reactive protein.

**Table 4B. Characteristics of age and sex matched sub-cohort, split by MHO and MUO**

| | **NW** | **MHO** | **MUO** | |
|---|---|---|---|---|
| | *n*=12 | *n*=7 | *n*=10 | P* |
| Age (years) | 41.25 ± 2.53 | 41.86 ± 3.55 | 41.70 ± 2.88 | 0.937 |
| Gender (M, F %) | 50,50 | 57,43 | 50,50 | 0.947 |
| BMI (kg/m²) | 23.64± 0.68^{a} | 50.60 ± 6.00^{b} | 45.40 ± 3.22^{b} | **<0.001** |
| SBP (mmHG) | 124.25 ± 3.78^{a} | 125.43 ± 2.79^{ab} | 137.60 ± 2.92^{b} | **0.017** |
| DBP (mmHG) | 75.42 ± 3.40^{a} | 80.86 ± 2.02^{ab} | 86.50 ± 2.91^{b} | **0.043** |
| TAG (mmol/L) | 1.02 ± 0.09^{a} | 1.01 ± 0.16^{a} | 2.21 ± 0.31^{b} | **<0.001** |
| Cholesterol (mmol/L) | 4.43 ± 0.19 | 4.32 ± 0.12 | 5.050 ± 0.42 | 0.187 |
| HDL (mmol/L) | 1.50 ± 0.10^{a} | 1.24 ± 0.10^{ab} | 1.03 ± 0.06^{b} | **0.002** |
| LDL (mmol/L) | 2.46 ± 0.24 | 2.68 ± 0.19 | 3.01 ± 0.31 | 0.315 |
| HbA1c (mmol/mol) | 29.34 ± 1.69^{a} | 38.41 ± 2.09^{ab} | 49.10 ± 8.64^{b} | **0.036** |
| Glucose (mmol/L) | 4.70 ± 0.09^{a} | 5.11 ± 0.18^{ab} | 6.83 ± 0.79^{b} | **0.008** |
| Insulin (mmol/L) † | 7.38±3.18^{a} | 28.64±5.54^{ab} | 28.90±6.24^{b} | **0.026** |
| HOMA-IR † | 1.43±1.41^{a} | 6.49±3.27^{ab} | 9.46±7.19^{b} | **0.006** |
| High sensitivity CRP (mg/L) † | 0.65±0.10^{a} | 8.60±4.12^{ab} | 9.29±2.74^{b} | **0.026** |

| | | | | |
|---|---|---|---|---|
| *n,* number of participants *Differences across groups were assessed using a one-way ANOVA with Bonferroni Correction ^{a,b} Mean values with unlike superscript letters are significantly different between groups (P<0.05) † *n*=6 (NW), *n*=7 (MHO), *n*=8 (MHO) , § *n*=9 (NW), *n*=5 (MHO), *n*=7 (MHO) | | | | |

Levels of 49 proteins were significantly different between MUO-HDL and NW-HDL (Figure 4A) with enrichment of complement factors (CF1, CO2, CO4B, CO6), acute phase response proteins (CRP, Serum amyloid P component (SAMP)) and coagulation factors (coagulation factor X and heparin cofactor 2 (Hep2)) and inter-alpha-trypsin inhibitors H1 (ITIH1), ITIH2 and ITIH4 on MUO-HDL relative to NW-HDL. By contrast significantly reduced levels of a range of immunoglobulins, apolipoproteins, PON1, gelsolin, alpha-2-antitrypsin and ceruloplasmin were observed on MUO-HDL relative to NW-HDL (Figure 4B-E). GO analysis of these differentially expressed proteins identified four major hubs; reverse cholesterol transport, regulation of protein processing (complement activation, acute phase response and negative regulation of blood coagulation), protein activation cascade and phagocytosis (Supplement Figure 3A&B).

### Example 4

### Differences in proteomic composition between MHO and MUO groups

The effect of metabolic health on HDL proteomic composition was subsequently assessed. A smaller number of proteins were identified as being significantly different between MHO and MUO groups (*n*=14) (Figure 5A). Coagulation factor X, angiotensinogen and kallikrein were enriched on MUO-HDL compared to MHO-HDL, while gelsolin, ceruloplasmin and a range of immunoglobulins were enriched on MHO-HDL compared to MUO-HDL (Figure 5B-D).

### Example 5

### Metabolic HDL Index (MHI) Score and correlation to the MetS

The HDL proteomic signature of NW and MUO groups could stratify individuals into their respective groups with 92% and 90% accuracy respectively. The MHO group by contrast exhibited greater variability in their HDL proteome with *n*=2 clustered with NW, *n*=3 clustered with MUO and *n*=2 falling into their own grouping. A scoring algorithm was generated based on significantly different proteins between NW and MUO groups. MHI decreased incrementally in MHO and MUO groups compared to NW (Figure 6A). Regression analysis was performed on the data-set generating the MHI and the list of contributing proteins is ranked in order of significance (Table 5). The top ten most significantly correlated proteins to MHI included CO6, CO4B, Hep2, protein IGKV2D-28, ApoC-I, ApoC-III, Ig heavy chain V-III region 23, ApoA-I, ApoA-IV, alpha-2-antiplasmin and gelsolin. The MHI score significantly correlated with BMI, glucose, systolic and diastolic blood pressure, HDL-C, fasting insulin and HOMA-IR with a trend towards negative correlation to TAG (p=0.056) (Figure 6B and Supplement Figure 4). A heat-map was generated based on the ranked MHI scores and clustered one MHO subject (BMI=30, MHI=17.7) into the NW group and another two MHO subjects (BMI=52, MHI= -25.4 and BMI=57, MHI= -22.8) into the MUO group (Figure 6C). Of interest one NW subject exhibited a MHI score (-7.5) more akin to the MHO group. These preliminary findings suggest that MHI could provide a novel continuous index upon which to measure metabolic health.

**Table 5. Linear Regression of MHI Score to 44 Proteins**

| | Gene | R | R² | P |
|---|---|---|---|---|
| Complement C4-B | CO4B | -0.797 | 0.635 | **<0.001** |
| Heparin cofactor 2 | HEP2 | -0.784 | 0.615 | **<0.001** |
| Protein IGKV2D-28 | IGKV2D-28 | 0.740 | 0.548 | **<0.001** |
| Apolipoprotein C-I | K7ERI9 | 0.735 | 0.540 | **<0.001** |
| Apolipoprotein C-III | APOC-III | 0.797 | 0.635 | **<0.001** |
| Ig heavy chain V-III region 23 | HV304 | 0.733 | 0.537 | **<0.001** |
| Apolipoprotein A-I | APOA-I | 0.777 | 0.604 | **<0.001** |
| Apolipoprotein A-IV | APOA-IV | 0.690 | 0.476 | **<0.001** |
| Alpha-2-antiplasmin | A2AP | 0.694 | 0.482 | **<0.001** |
| Gelsolin | GELS | 0.682 | 0.465 | **<0.001** |
| Complement factor I | G3XAM2 | -0.636 | 0.404 | **<0.001** |
| Protein IGHV4-31 | IGHV4-31 | 0.649 | 0.421 | **<0.001** |
| Ceruloplasmin | CERU | 0.633 | 0.401 | **<0.001** |
| Serum paraoxonase/arylesterase 1 | PON1 | 0.610 | 0.372 | **<0.001** |
| Apolipoprotein D | APOD | 0.669 | 0.448 | **<0.001** |
| Ig lambda-3 chain C regions | IGLC3 | 0.576 | 0.332 | **0.001** |
| Immunoglobulin lambda variable 2-8 | IGLV2-8 | 0.574 | 0.329 | **0.001** |
| Complement C2 | CO2 | -0.572 | 0.327 | **0.001** |
| Inter-alpha-trypsin inhibitor heavy chain H4 | ITIH4 | -0.585 | 0.342 | **0.001** |
| Ig kappa chain C region | IGKC | 0.582 | 0.339 | **0.001** |
| Inter-alpha-trypsin inhibitor heavy chain H2 | Q5T985 | -0.543 | 0.295 | **0.002** |
| Ig gamma-2 chain C region | IGHG2 | 0.522 | 0.272 | **0.002** |
| Uncharacterized protein | B4E1Z4 | -0.530 | 0.281 | **0.003** |
| Protein IGKV1-17 | IGKV1-17 | 0.540 | 0.292 | **0.004** |
| C-reactive protein | CRP | 0.518 | 0.268 | **0.004** |
| Leucine-rich alpha-2-glycoprotein | A2GL | -0.505 | 0.255 | **0.005** |
| Complement component C6 | CO6 | -0.516 | 0.266 | **0.005** |
| Coagulation factor X | FA10 | -0.495 | 0.245 | **0.006** |
| Protein IGKV1-33 | IGKV1D-33 | 0.500 | 0.250 | **0.006** |
| Inter-alpha-trypsin inhibitor heavy chain H1 | ITIH1 | -0.495 | 0.245 | **0.006** |
| Sex hormone-binding globulin | I3L145 | 0.478 | 0.228 | **0.009** |
| Ig heavy chain V-I region 5 | KV110 | 0.503 | 0.253 | **0.009** |
| Serum amyloid P-component | SAMP | -0.465 | 0.216 | **0.011** |
| Vitamin K-dependent protein S | PROS | -0.508 | 0.258 | **0.013** |
| Ig gamma-1 chain C region | IGHG1 | 0.455 | 0.207 | **0.013** |
| Phosphatidylinositol-glycan-specific phospholipase D | PHLD | -0.463 | 0.214 | **0.015** |
| Plasma protease C1 inhibitor | IC1 | -0.415 | 0.172 | **0.025** |
| Ig lambda chain V-I region 51 | LV104 | 0.421 | 0.177 | **0.032** |
| Actin, cytoplasmic 2 | ACTG | -0.415 | 0.172 | **0.032** |
| Protein IGHV3-74 | IGHV3-74 | -0.366 | 0.134 | 0.051 |
| Corticosteroid-binding globulin | CBG | 0.356 | 0.127 | 0.058 |
| Protein IGKV2D-29 | IGKV2D-29 | -0.373 | 0.139 | 0.067 |
| Vitamin K-dependent protein C | PROC | -0.317 | 0.100 | 0.094 |
| Ig lambda chain V-II region BUR | LV205 | -0.286 | 0.082 | 0.140 |

HDL particle functionality has emerged as a novel target and more important determinant of cardiovascular risk than static HDL-C levels. Pathway analysis of the HDL proteome has identified HDL particle remodelling, acute inflammatory response, protein activation cascades, and reverse cholesterol transport as the major pathways associated with the particles which mirror the assigned cardio-protective functions of HDL. The alignment of HDL protein pathways with particle functions suggests that protein composition of HDL is not only specific, but is fundamental, to biological effects. The present invention has identified important changes in the network of proteins associating with HDL in obese subjects compared to NW controls (49 out of 146 proteins significantly changed) with enrichment of pro-inflammatory acute phase proteins and loss of anti-oxidant/anti-inflammatory proteins on obese particles. These findings indicate that HDL particles become metabolically activated during obesity with decreased cardio-protective potential. Further to this, the present invention demonstrates reduced PON-1 activity and reduced ABCA1-independent efflux capacity of serum in obese subjects compared to NW controls. Increasing the 'quality', as opposed to the quantity, of HDL particles in turn might be more beneficial in the setting of obesity.

While obesity increases the risk of CVD, this risk is enhanced with concurrent presentation of the MetS. The present invention therefore explores whether metabolic health status is an important prerequisite for the preservation of healthy HDL particles in the obese state. Chronic inflammation is a classic hallmark of obesity that contributes to development of insulin resistance and likely is the causal link for enhanced cardiovascular risk. Without being bound by theory, the inventors therefore speculated that the sub-acute chronic inflammation observed in MUO may exaggerate HDL dysfunction and proteomic composition.

The inventors have evaluated total, ABCA1-independent and ABCA1-dependent efflux capacity of serum to delineate the ability of total, large and small HDL particles to support efflux respectively in NW, MHO and MUO groups; and demonstrate reduced total efflux capacity of serum from obese individuals compared to NW control, which was attributable to a specific reduction in ABCA1-independent efflux and not ABCA1-dependent efflux. FPLC analysis demonstrated reduced cholesterol within larger HDL fractions from obese individuals compared to NW, indicative that the number of larger HDL particles, the main acceptors via ABCA1-independent pathways, is reduced. Indeed, normalisation of results to HDL-C input demonstrates that HDL-C is an important determinant of reduced ABCA1-independent efflux in obese subjects. No significant difference in HDL efflux capacity was evident between MHO and MUO sub-groups.

PON1 is an important anti-oxidant protein that is primarily carried on HDL in serum and reduced levels are associated with increased CVD risk. The inventors hence measured serum PON1 activity as a surrogate for measuring the anti-oxidant capacity of HDL particles. Serum PON1 activity was significantly reduced in the obese cohort compared to NW - again no significant difference was observed between MHO and MUO sub-groups. Lack of difference in HDL functionality between MHO and MUO groups suggests that the obese phenotype alone is sufficient to drive HDL dysfunction or that stratification of obese individuals based on presence or absence of the MetS is not sensitive enough to distinguish between cohorts.

The efflux capacity of isolated HDL-fractions was evaluated *ex vivo* and demonstrated reduced efflux to the larger HDL fraction in the obese group compared to NW, with no difference in efflux to small/medium fractions, which was consistent with findings in ApoB-depleted serum. Levels of pro-inflammatory SAA1 were enriched on smaller obese-HDL particles compared to NW-HDL indicative of a pro-inflammatory particle, an effect that is also evident in patients with type 1 diabetes. FPLC analysis demonstrated reduced cholesterol on larger HDL fractions (fractions 30-36), with preservation of cholesterol on smaller HDL fractions (fractions 36-40), and increased LDL-C levels in the obese cohort compared to NW. Proteomic profiling of HDL particles was performed on HDL fraction 38, where HDL-C was equivalent between groups to avoid introduction of a potential systematic error. Furthermore, the obese group was sub-divided into MHO and MUO groupings to establish whether HDL proteomics was more sensitive to detect differences between these groups than HDL function assays.

A remarkable difference in the HDL proteome was evident between age- and sex-matched NW and MUO groups. Indeed, blinded analysis of the proteomics data could accurately separate the NW (91.7%, 11/12) and MUO (90%, 9/10) groups. MUO HDL particles were enriched for complement factor I, complement C4B, C2 and C6, C-reactive protein, serum amyloid P-component, heparin cofactor 2 (Hep2) and coagulation factor X. By contrast, Apo- AI, AIV, CI, CIII and D, paraoxonase, ceruloplasmin, sex hormone binding globulin (SHBG), cortiocosteroid binding globulin, and alpha-2-antiplasmin were all significantly reduced on MUO-HDL compared to NW-HDL. Previous plasma proteomics investigating the effects of weight-loss in obese individuals on the plasma proteome and demonstrated a specific reversal in some of the parameters observed in our study with down regulation of CRP and Hep2 and upregulation of SHBG. The inventors have observed a significant reduction in ApoC-III on MUO-HDL particles that strongly correlated with ApoA-I (r=0.89), despite plasma levels of ApoC-III usually being elevated with the MetS and CVD. ApoC-III prevents efficient catabolism of triglyceride-rich lipoprotein particles and is hence associated with hypertriglyceridemia. Sequestering of ApoC-III from ApoB/chylomicrons onto HDL improves triglyceride clearance in circulation and hence the re-direction of ApoC-III from HDL onto other triglyceride-rich lipoproteins within the obese cohort could partially mediate hypertriglyceridemia. Enrichment of MUO particles with pro-inflammatory proteins and loss of anti-inflammatory/anti-oxidant proteins indicates presence of a metabolically activated HDL particle within the MUO setting.

HDL proteomic analysis within the MHO sub-group revealed a greater diversity in expression with prediction tools placing 2/7 individuals in the NW group, 3/7 within the MUO group and 2/7 within their own grouping. These results are consistent with the growing evidence that many MHO individuals eventually progress into the MUO category and indeed HDL proteomics was able to identify a number of individuals who are likely at higher risk due to their metabolic HDL profile. A number of significantly different proteins (*n*=14) were noted between MHO and MUO HDL proteomes; coagulation FX, kallikrein and angiotensinogen were upregulated on MUO-HDL compared to MHO-HDL and are involved in the coagulation cascade, fibrinolysis and control of blood pressure. By contrast gelsolin, ceruloplasmin and a range of immunoglobulins were increased on MHO-HDL compared to MUO-HDL.

Given the accuracy of proteomic data to predict grouping of NW and MUO individuals, the present invention relates to a scoring algorithm to generate a metabolic HDL index (MHI). The MHI score positively correlated with HDL-C and ABCA1 -independent efflux and negatively correlated with hypertension, hyperglycaemia, BMI, fasting insulin and HOMA-IR. Interestingly when the total cohort was unclustered and re-grouped based on MHI, we identified one MHO individual (BMI=30.2) who exhibited a MHI score akin to the NW group, while two MHO individuals (BMI = 52 and 57) exhibited a MHI score that would re-classify them as MUO. We also identified one NW subject with a MHI score that aligned with the MHO group. These preliminary results suggest that HDL proteomic analysis could provide more sensitive stratification of high-risk lean and obese individuals than currently used guidelines but this remains to be validated.

The present invention has established a metabolic HDL index score based on HDL proteomic composition that correlates with metabolic health status and may provide a useful tool for more accurate stratification of high-risk individuals and subsequent assignment to more aggressive interventions as warranted.

## Claims

1. A method of diagnosing or prognosing a metabolic disorder selected from one or more of abdominal obesity, high blood pressure (hypertension), high blood sugar (hyperglycaemia), high serum triglycerides (hypertriglyceridemia), and low high-density lipoprotein (HDL) levels (HDL-cholesterol(C)) in a subject, the method comprising the steps of:
(a) determining the quantitative or qualitative level of the protein biomarkers in a biological sample from the subject; and
(b) diagnosing or prognosing the metabolic disorder in the subject based on the quantitative or qualitative level of each protein biomarker in the biological sample;
wherein the protein biomarkers comprise Complement C4-B; Heparin cofactor 2; Protein IGKV2D-28; Apolipoprotein C-I; Apolipoprotein C-III; Ig heavy chain V-III region 23; Apolipoprotein A-I; Apolipoprotein A-IV; Alpha-2-antiplasmin; and Gelsolin.

2. The method of claim 1, wherein the protein biomarkers further comprise: Complement factor I; Protein IGHV4-31; Ceruloplasmin; Serum paraoxonase/arylesterase 1; Apolipoprotein D; Ig lambda-3 chain C regions; Immunoglobulin lambda variable 2-8; Complement C2; Inter-alpha-trypsin inhibitor heavy chain H4; Ig kappa chain C region; Inter-alpha-trypsin inhibitor heavy chain H2; Ig gamma-2 chain C region; Uncharacterized protein B4E1Z4; Protein IGKV1-17; and C-reactive protein cleaved into: C-reactive protein(1-205).

3. The method of claim 2, wherein the protein biomarkers further comprise: Leucine-rich alpha-2-glycoprotein; Complement component C6; Coagulation factor X; Protein IGKV1-33; Inter-alpha-trypsin inhibitor heavy chain H1; Sex hormone-binding globulin; Ig heavy chain V-I region 5; Serum amyloid P-component; Vitamin K-dependent protein S; Ig gamma-1 chain C region; Phosphatidylinositol-glycan-specific phospholipase D; Plasma protease C1 inhibitor; Ig lambda chain V-I region 51;Actin, cytoplasmic 2; Protein IGHV3-74; Corticosteroid-binding globulin; Protein IGKV2D-29; Vitamin K-dependent protein C; and Ig lambda chain V-II region BUR.

4. The method of any one of claims 1-3, wherein the method is a method of diagnosing or prognosing the metabolic disorder in the subject, irrespective of the weight of the subject.

## Patentansprüche

1. Verfahren zum Diagnostizieren oder Prognostizieren einer Stoffwechselerkrankung, ausgewählt aus einem oder mehreren von abdominaler Fettleibigkeit, Bluthochdruck (Hypertonie), hohem Blutzucker (Hyperglykämie), hohen Serumtriglyceriden (Hypertriglyceridämie) und niedrigen Spiegeln von Lipoprotein hoher Dichte (high-density-lipoprotein - HDL) (HDL-Cholesterin(C)) bei einem Subjekt, wobei das Verfahren die folgenden Schritte umfasstm
(a) Bestimmen des quantitativen oder qualitativen Niveaus der Proteinbiomarker in einer biologischen Probe von dem Subjekt; und
(b) Diagnostizieren oder Prognostizieren der Stoffwechselerkrankung bei dem Subjekt auf Grundlage des quantitativen oder qualitativen Niveaus jedes Proteinbiomarkers in der biologischen Probe;
wobei die Proteinbiomarker Komplement C4-B; Heparin-Cofaktor 2; Protein IGKV2D-28; Apolipoprotein C-I; Apolipoprotein C-III; V-III-Region 23 der schweren Ig-Kette; Apolipoprotein A-I; Apolipoprotein A-IV; Alpha-2-Antiplasmin und Gelsolin umfassen.

2. Verfahren nach Anspruch 1, wobei die Proteinbiomarker ferner umfassen: Komplementfaktor I; Protein IGHV4-31; Ceruloplasmin; Serum-Paraoxonase/Arylesterase 1; Apolipoprotein D; C-Regionen der Ig-Lambda-3-Kette; Immunglobulin lambda variable 2-8; Komplement C2; schwere Kette H4 des Inter-Alpha-Trypsin-Inhibitors; C-Region der Ig-Kappa-Kette; schwere Kette H2 des Inter-Alpha-Trypsin-Inhibitors; C-Region der lg-Gamma-2-Kette; uncharakterisiertes Protein B4E1Z4; Protein IGKV1-17 und C-reaktives Protein gespalten in: C-reaktives Protein (1-205).

3. Verfahren nach Anspruch 2, wobei die Proteinbiomarker ferner umfassen: Leucin-reiches Alpha-2-Glykoprotein; Komplementkomponente C6; Gerinnungsfaktor X; Protein IGKV1-33; schwere Kette H1 des Inter-Alpha-Trypsin-Inhibitors; Sexualhormon-bindendes Globulin; V-I-Region 5 der schweren Ig-Kette; Serum-Amyloid-P-Komponente; Vitamin K-abhängiges Protein S; C-Region der Ig-Gamma-1-Kette; Phosphatidylinosit-Glykan-spezifische Phospholipase D; Plasma-Protease-C1-Inhibitor; V-I-Region 51 der Ig-Lambda-Kette; Aktin, zytoplasmatisch 2; Protein IGHV3-74; Corticosteroid-bindendes Globulin; Protein IGKV2D-29; Vitamin K-abhängiges Protein C und V-II-Region BUR der Ig-Lambda-Kette.

4. Verfahren nach einem der Ansprüche 1-3, wobei das Verfahren ein Verfahren zum Diagnostizieren oder Prognostizieren der Stoffwechselerkrankung bei dem Subjekt, unabhängig von dem Gewicht des Subjekts, ist.

## Revendications

1. Procédé de diagnostic ou de pronostic d'un trouble métabolique choisi parmi un ou plusieurs parmi l'obésité abdominale, l'hypertension artérielle, l'hyperglycémie, les triglycérides sériques élevés (hypertriglycéridémie) et les niveaux de lipoprotéine de haute densité (HDL) faibles (HDL-cholestérol(C)) chez un sujet, le procédé comprenant les étapes de :
(a) détermination du niveau quantitatif ou qualitatif des biomarqueurs de protéines dans un échantillon biologique du sujet ; et
(b) diagnostic ou pronostic du trouble métabolique chez le sujet sur la base du niveau quantitatif ou qualitatif de chaque biomarqueur de protéine dans l'échantillon biologique ; dans lequel les biomarqueurs de protéines comprennent le complément C4-B ; le cofacteur d'héparine 2 ; la protéine IGKV2D-28 ; l'apolipoprotéine C-I ; l'apolipoprotéine C-III ; la région 23 de chaîne lourde Ig V-III ; l'apolipoprotéine AI ; l'apolipoprotéine A-IV ; l'Alpha-2-antiplasmine ; et la gelsoline.

2. Procédé selon la revendication 1, dans lequel les biomarqueurs de protéines comprennent en outre : le complément facteur I ; la protéine IGHV4-31 ; la céruloplasmine ; la paraoxonase sérique/l'arylestérase 1 ; l'apolipoprotéine D ; les régions C de la chaîne Ig lambda-3 ; l'immunoglobuline lambda variable 2-8 ; le complément C2 ; la chaîne lourde H4 de l'inhibiteur de l'inter-alpha-trypsine ; la région C de la chaîne kappa Ig ; la chaîne lourde H2 de l'inhibiteur de l'inter-alpha-trypsine ; la région C de la chaîne gamma-2 Ig ; la protéine non **caractérisée** B4E1Z4 ; la protéine IGKV1-17 ; et la protéine C-réactive clivée en : protéine C-réactive (1-205) .

3. Procédé selon la revendication 2, dans lequel les biomarqueurs de protéines comprennent en outre : l'alpha-2-glycoprotéine riche en leucine ; le composant complémentaire C6 ; le facteur de coagulation X ; la protéine IGKV1-33 ; la chaîne lourde H1 de l'inhibiteur de l'inter-alpha-trypsine ; la globuline liant les hormones sexuelles ; la région 5 de la chaîne lourde Ig V-I ; le composant P amyloïde sérique ; la protéine S dépendante de la vitamine K ; la région C de la chaîne gamma-1 Ig ; la phospholipase D spécifique du phosphatidylinositol-glycane ; l'inhibiteur de la protéase plasmatique C1 ; la région 51 de la chaîne lambda Ig V-I ; l'actine, cytoplasmique 2 ; la protéine IGHV3-74 ; la globuline liant les corticostéroïdes ; la protéine IGKV2D-29 ; la protéine C dépendante de la vitamine K ; et la région BUR de la chaîne lambda Ig V-II.

4. Procédé selon l'une quelconque des revendications 1 à 3, dans lequel le procédé est un procédé de diagnostic ou de pronostic du trouble métabolique chez le sujet, quel que soit le poids du sujet.
